# EUROPEAN PATENT SPECIFICATION

(11) **EP 0 607 332 B1**
(45) Date of publication and mention of the grant of the patent: **17.12.1997**
(21) Application number: 92922246.1
(22) Date of filing: 06.10.1992
(51) Int. Cl.: A61K 39/395, A61K 39/02

(54) **METHOD OF PROPHYLAXIS OR TREATMENT OF ANTIGEN PRESENTING CELL DRIVEN SKIN CONDITIONS USING INHIBITORS OF THE CD2/LFA-3 INTERACTION**
VORBEUGUNGSMETHODE ODER BEHANDLUNG VON, DURCH ANTIGEN-PRÄSENTIERENDE ZELLEN HERVORGERUFENE, HAUTKRANKHEITEN MITTELS INHIBITOREN DER CD2/LFA-3 WECHSELWIRKUNG
PROCEDE PROPHYLACTIQUE OU THERAPEUTIQUE DE MALADIES DE LA PEAU CAUSEES PAR DES CELLULES PRESENTANT DES ANTIGENES AU MOYEN D'INHIBITEURS DE L'INTERACTION ENTRE CD2 ET LFA-3

(30) Priority: 07.10.1991 US 770969; 02.04.1992 US 862022
(43) Date of publication of application: 27.07.1994
(73) Proprietor: BIOGEN, INC., Cambridge Massachusetts 02142 (US); THE REGENTS OF THE UNIVERSITY OF MICHIGAN, Ann Arbor, Michigan 48109-1248 (US)
(72) Inventor: WALLNER, Barbara, P., Cambridge, MA 02139 (US); COOPER, Kevin, D., Ann Arbor, MI 48105 (US)
(74) Representative: Ruffles, Graham Keith
(86) International application number: US9208755
(87) International publication number: WO9306866

(56) References cited:
- TRANSPLANTATION, vol. 51, no. 1, January 1991, Baltimore, MD (US); J. BROMBERG et al., pp. 219-225
- ARTHRITIS & RHEUMATISM, vol. 34, no. 9, September 1991, New York, NY (US); D. ABRAHAM et al., pp. 1164-1172
- EXPERIMENTAL CELL RESEARCH, vol. 190, no. 1, September 1990, New York, NY (US); D. ABRAHAM et al., pp. 118-126
- CLINICAL RESEARCH, vol. 40, no. 2, 1992, Thorofare, NJ (US); A. GONZALES-RAMOS et al., p. 500A

## Description

### TECHNICAL FIELD OF THE INVENTION

This invention relates to methods of using inhibitors of the CD2/LFA-3 interaction in treating skin conditions characterized by increased T cell activation and abnormal antigen presentation in the dermis and epidermis in mammals, including humans. Such conditions include psoriasis, UV damage, atopic dermatitis, cutaneous T cell lymphoma such as mycosis fungoides, allergic and irritant contact dermatitis, lichen planus, alopecia areata, pyoderma gangrenosum, vitiligo, ocular cicatricial pemphigoid, and urticaria.

### BACKGROUND OF THE INVENTION

There are numerous skin conditions characterized by increased T cell activation and abnormal antigen presentation in the dermis and epidermis. The pathophysiologic mechanisms involved in the evolution of such inflammatory processes are poorly understood. However, it has become apparent that skin cells are important in the generation of a cutaneous inflammatory response (Kupper, "Immune and Inflammatory Processes in Cutaneous Tissues", J. Clin. Invest., 86, pp. 1783-89 (1990)).

The normal adult epidermal population contains 1-2% Langerhans' cells and about 98% keratinocytes. Keratinocytes and other nonhematopoietically-derived cells resident in skin contribute to immune homeostasis and can produce various cytokines which influence migration of T cells and expression of adhesion molecules.

As antigen presenting cells, Langerhans' cells express a high density of Class II major histocompatibility complex (MHC) antigen on the cell surface. MHC Class II molecules bind peptides derived from endocytosed antigen and are recognized primarily by helper T lymphocytes. The T cell receptor on T cells recognizes antigen as a peptide fragment bound to the cell-surface molecules encoded by the MHC (Springer, "Adhesion Receptors of the Immune System", Nature, 346, pp. 425-27 (1990)).

There are many interactions between molecules expressed on the surface of Langerhans' cells and the surface of T cells, in addition to the T cell receptor/MHC interaction. These surface molecules, often referred to as adhesion molecules, participate in a number of functions including cellular adhesion, antigen recognition, co-stimulatory signalling in T cell activation and stimulation of effectors of T cell cytotoxicity ("Adhesion Molecules in Diagnosis and Treatment of Inflammatory Diseases", The Lancet, 336, pp. 1351-52 (1990)). Such cell adhesion appears to be involved in activation of T cell proliferation in the generation of an immune response (Hughes et al., "The Endothelial Cell as a Regulator of T-cell Function", Immunol. Rev., 117, pp. 85-102 (1990)).

Various skin conditions are characterized by increased T cell activation and abnormal antigen presentation in the dermis and epidermis (Cooper, "Immunoregulation in the Skin", in Cutaneous Lymphoma, Curr. Probl. Dermatol., eds. van Vloten et al., 19, pp. 69-80 at pp. 73, 74, 76 (1990)). For example, in contact allergic dermatitis, activation of intracutaneous T cells is observed. It is known that skin from patients exhibiting atopic dermatitis contains an increased number of Langerhans' cells (Cooper, "Immunoregulation in the Skin", in Cutaneous Lymphoma, Curr. Probl. Dermatol., eds. van Vloten et al., 19, at p. 74 (1990)). In psoriatic skin, there is an increased number of antigen presenting cells, composed of both Langerhans' cells and non-Langerhans' cell Class II MHC-bearing antigen presenting cells (Cooper, "Immunoregulation in the Skin", in Cutaneous Lymphoma, Curr. Probl. Dermatol., eds. van Vloten et al., 19, at p. 75 (1990)).

UV exposed skin is characterized by an overall depletion of Langerhans' cells and migration of a non-Langerhans' cell antigen-presenting cell population into the epidermis, which activates autologous T cells to proliferate (Cooper, "Immunoregulation in the Skin" in Cutaneous Lymphoma, Curr. Probl. Dermatol., eds. van Vloten et al., 19, at pp. 75-76 (1990)). In human skin after 4 minimal erythemal doses of UV B, Langerhans' cells (the constitutive antigen presenting cell population) are inactivated for approximately 3 days (Cooper et al., "Effects Of Ultraviolet Radiation On Human Epidermal Cell Alloantigen Presentation: Initial Depression Of Langerhans Cell-Dependent Function Is Followed By Appearance Of T6-DR⁺ Cells That Enhance Epidermal Alloantigen Presentation", J. Immunol., 134, pp. 129-37 (1985)). In this type of UV damaged skin, the CD1a⁻DR⁺ macrophage population (a population of antigen presenting cells) increases from 0% (normal skin) to approximately 2-10% of the entire epidermal cell population and is the cell population entirely responsible for the induction of T cell proliferation to alloantigen. (Cooper et al., J. Immunol., supra (1985); Baadsgaard et al., "In Vivo Ultraviolet-Exposed Human Epidermal Cells Activate T Suppressor Cell Pathways That Involve CD4⁺ CD45RA⁺ Suppressor-Inducer T cells", J. Immunol., 145, pp. 2854-61 (1990)).

Cutaneous T cell lymphoma is characterized by the expansion of a malignant clonal population of T cells in the dermis and epidermis. Lesional epidermal cells contain increased numbers of CD1⁺ DR⁺ antigen presenting cells (Cooper, "Immunoregulation in the Skin" in Cutaneous Lymphoma, Curr. Probl. Dermatol., eds. van Vloten et al., 19, at pp. 76-77 (1990)).

Presently known therapies for the above mentioned skin diseases are inadequate. Steroids or cyclosporin A are commonly used in the treatment of psoriasis, lichen planus, urticaria, atopic dermatitis, UV damage, pyoderma gangrenosum, vitiligo, ocular cicatricial pemphigoid, alopecia areata, allergic and irritant contact dermatitis and cutaneous T cell lymphoma. In addition, for some of these skin conditions, various therapies include retinoids, PUVA, nitrogen mustard, interferon, chemotherapy, methotrexate, UV light, antibiotics and antihistamines. See generally Fitzpatrick, Dermatology in General Medicine, 3rd Ed., McGraw Hill (1987).

Side effects to these therapies are known. Most commonly encountered drawbacks for cyclosporin A include toxicity due to immunosuppression and renal and neural toxicity. Steroids have well known side effects including induction of Cushing Syndrome. Side effects of certain of the other aforementioned therapies include skin cancer, bone marrow and constitutional toxicities, ligament calcification, liver fibrosis and other disorders.

T cells play a major role in the immune response by interacting with target and antigen presenting cells. For example, T cell-mediated killing of target cells is a multi-step process involving, initially, adhesion of cytolytic T cells (the effector cells) to target cells. Also, helper T cells help initiate the immune response by adhesion to antigen presenting cells.

These interactions of T cells with target and antigen presenting cells are highly specific and depend on the recognition of an antigen on the surface of a target or antigen presenting cell by one of the many specific antigen receptors on the surface of T cells.

The receptor-antigen interaction of T cells and other cells is also facilitated by various T cell surface proteins, e.g., the antigen-receptor complex CD3 and accessory adhesion molecules such as CD4, LFA-1, CD8, and CD2. It is also facilitated by accessory adhesion molecules, such as LFA-3, ICAM-1 and MHC, that are expressed on the surface of the target or antigen presenting cells. For example, LFA-1 and its counter receptor ICAM-1 or ICAM-2, as well as CD2 and its counter receptor LFA-3 have been implicated in cellular adhesion and T cell activation. It is known that the LFA-1/ICAM and CD2/LFA-3 interactions are independent.

A number of other molecules present on resting T cells have also been implicated in T cell adhesion, including E2 (MIC2), VLA-4 (CD49d), CD44 (Hermes, Pgp-1, ECMRIII), and H19 (N4) (see Makgoba et al., "The CD2-LFA-3 and LFA-1-ICAM Pathways: Relevance to T-cell Recognition", Immunol. Today, 10, pp. 417-22 (1989)).

One way in which T cells are activated is by binding of their antigen specific T cell receptors to peptide-MHC complexes on the surface of antigen presenting cells such as macrophages. T cell activation stimulates proliferation and differentiation of two types of functional T cells: helper cells, which promote the proliferation and maturation of antibody-producing B lymphocytes, and killer cells, which lyse target cells (Bierer et al., "A Monoclonal Antibody to LFA-3, the CD2 Ligand, Specifically Immobilizes Major Histocompatibility Complex Proteins", Eur. J. Immunol. 19, pp. 661-65 (1989); Springer "Adhesion Receptors of the Immune System", Nature, 346, pp. 425-34 (1990)).

The interaction between CD2 and LFA-3 remains poorly understood with respect to activation of T cell activity. Recent studies have suggested that there is a specific interaction between CD2 (a T cell adhesion molecule) and LFA-3 (a target cell and antigen presenting cell adhesion molecule) which mediates T cell adhesion to the target or antigen presenting cells. This cell-cell adhesion has been implicated in the initiation of T cell functional responses (Dustin et al., "Purified Lymphocyte Function Associated Antigen 3 Binds to CD2 and Mediates T Lymphocyte Adhesion," J. Exp. Med., 165, pp. 677-92 (1987); Springer et al., "The Lymphocyte Function-associated LFA-1, CD2, and LFA-3 Molecules: Cell Adhesion Receptors of the Immune System", Ann. Rev. Immunol., 5, pp. 223-52 (1987)).

LFA-3, which is found on the surface of a wide variety of cells, including human erythrocytes, has become the subject of a considerable amount of study to further elucidate its role in various T cell interactions (see, e.g., Krensky et al., "The Functional Significance, Distribution, and Structure of LFA-1, LFA-2, and LFA-3: Cell Surface Antigen Associated with CTL-Target Interactions", J. Immunol., 131(2), pp. 611-16 (1983); Shaw et al., "Two Antigen-Independent Adhesion Pathways Used by Human Cytotoxic T-cell Clones", Nature, 323, pp. 262-64 (1986)). Two natural forms of LFA-3 have been identified. One form of LFA-3 ("transmembrane LFA-3") is anchored in the cell membrane by a transmembrane hydrophobic domain. cDNA encoding this form of LFA-3 has been cloned and sequenced (see, e.g., Wallner et al., "Primary Structure of Lymphocyte Function-Associated Antigen-3 (LFA-3)", J. Exp. Med., 166, pp. 923-32 (1987)). Another form of LFA-3 is anchored to the cell membrane via a covalent linkage to phosphatidylinositol ("PI")-containing glycolipid. This latter form has been designated "PI-linked LFA-3", and cDNA encoding this form of LFA-3 has also been cloned and sequenced (Wallner et al., PCT publn. WO 90/02181).

The human CD2 (T11) molecule is a 50 kD surface glycoprotein expressed on >95% of thymocytes and virtually all peripheral T lymphocytes. Biochemical analyses using specific monoclonal antibodies have suggested that CD2 is T lineage-specific and exists on the cell surface in several differentially glycosylated forms (Howard et al., "A Human T Lymphocyte Differentiation Marker Defined by Monoclonal Antibodies that Block E-Rosette Formation", J. Immunol., 126, pp. 2117-22 (1981); Brown et al., in Leukocyte Typing III, ed. McMichael, Oxford University Press, pp. 110-12 (1987); Sayre et al., "Molecular Cloning and Expression of T11 cDNAs Reveals a Receptor-Like Structure on Human T Lymphocytes", Proc. Natl. Acad. Sci. USA, 84, pp. 2941-45 (1987)).

The sequence of a human CD2 gene has been reported (Seed and Aruffo, "Molecular Cloning of the CD2 Antigen, the T-cell Erythrocyte Receptor, by a Rapid Immunoselection Procedure", Proc. Natl. Acad. Sci. USA, 84, pp. 3365-69 (1987); Sayre et al., "Molecular Cloning and Expression of T11 cDNAs Reveal a Receptor-like Structure on Human T Lymphocytes", Proc. Natl. Acad. Sci. USA, 84, pp. 2941-45 (1987)). CD2 cDNA clones predict a cleaved signal peptide of 24 amino acid residues, an extracellular segment of 185 residues, a transmembrane domain of 25 residues and a cytoplasmic region of 117 residues (Sayre et al., supra (1987); Sewell et al., "Molecular Cloning of the Human T-Lymphocyte Surface CD2 (T11) Antigen", Proc. Natl. Acad. Sci. USA, 83, pp. 8718-22 (1986); Seed and Aruffo, supra (1987); Clayton et al., Eur. J. Immunol., 17, pp. 1367-70 (1987)).

Soluble CD2 polypeptides having an LFA-3 binding domain have been reported (PCT publ. WO 90/08187).

Monoclonal antibodies to CD2, for example TS2/18, T11₁, T11₂, T11₃, and to LFA-3, for example TS2/9, have also been reported (see, e.g., Hughes et al., "The Endothelial Cell as a Regulator of T-Cell Function", Immunol. Reviews, 117, pp. 85-102 (1990); Meuer, "An Alternative Pathway of T-Cell Activation: A Functional Role for the 50 kd T11 Sheep Erythrocyte Receptor Protein", Cell, 36, pp. 897-906 (1984)).

The role of antimurine CD2 mAb in the murine response was investigated by Bromberg et al., "Anti-CD2 Monoclonal Antibodies Alter Cell-Mediated Immunity In Vivo", Transplantation, 51, pp 219-225 (1991).

Soluble LFA-3 polypeptides having a CD2 affinity form part of the subject of a Ph.D. thesis entitled "Genetic Analysis of CD2/LFA-3 and CD4/HIV interactions" by Andrew Scott Peterson, Department of Genetics, Harvard University (July 1988).

The need still exists for improved methods of preventing and treating skin conditions exhibiting increased T cell activation and abnormal antigen presentation.

### SUMMARY OF THE INVENTION

The present invention provides the use of an inhibitor of the CD2/LFA-3 interaction for the manufacture of a medicament for preventing or treating human skin conditions characterized by increased T cell activation and abnormal antigen presentation in the dermis and epidermis.

The present invention also provides an inhibitor of the CD2/LFA-3 interaction which is a polypeptide consisting of a soluble LFA-3 polypeptide linked to an immunoglobulin hinge and heavy chain constant region or portions thereof, for use in a method of therapy practised on the human or animal body.

In the following discussion, a reference to a method of this invention is a reference to the underlying method for preventing or treating human skin conditions in which the medicament manufactured by the use of this invention can be employed, or, as appropriate, a reference to the underlying method of therapy in which the polypeptide consisting of a soluble LFA-3 polypeptide linked to an immunoglobulin hinge and heavy chain constant region or portions thereof can be employed.

The present invention generally solves many of the problems referred to above. It for the first time provides a method of preventing or treating skin conditions, characterized by increased T cell activation and abnormal antigen presentation in the dermis and epidermis, in a mammal, whereby an inhibitor of the CD2/LFA-3 interaction is administered to the mammal. The methods of this invention are superior to previously available therapies for these skin conditions for many reasons, including less immunosuppression than pre-existing therapies and more specific therapy with less general toxicity.

The method of the present invention preferably will be used in the treatment or prophylaxis of skin conditions selected from psoriasis, UV damage, atopic dermatitis, cutaneous T cell lymphoma such as mycosis fungoides, allergic and irritant contact dermatitis, lichen planus, alopecia areata, pyoderma gangrenosum, vitiligo, ocular cicatricial pemphigoid, and urticaria, preferably psoriasis or UV damage.

Inhibitors that can be used in accordance with the method of the present invention include any molecule that inhibits the CD2/LFA-3 interaction. Preferably, the inhibitor is selected from the group consisting of anti-LFA-3 antibody homologs, anti-CD2 antibody homologs, soluble LFA-3 polypeptides, soluble CD2 polypeptides, CD2 or LFA-3 mimetic agents and derivatives thereof.

### BRIEF DESCRIPTION OF THE DRAWINGS

Figure 1 illustrates the percent inhibition caused by an anti-LFA-3 monoclonal antibody (7A6) or an anti-CD2 monoclonal antibody (TS2/18) as compared to a non-specific control IgG₁ antibody (MOPC21) of autologous T cell activation by psoriatic epidermal cells in 4 patients.

Figure 2 illustrates the inhibition of allogeneic T cell activation by UV damaged epidermal cells ([³H]TdR incorporation) caused by an anti-LFA-3 monoclonal antibody (1E6) or an anti-CD2 monoclonal antibody (TS2/18) as compared to a non-specific IgG₁ antibody (MOPC21).

### DETAILED DESCRIPTION OF THE INVENTION

### Definitions

As used herein, "CD2" means a CD2 polypeptide that binds to a naturally occurring LFA-3 polypeptide and which is encoded by (a) a naturally occurring mammalian CD2 DNA sequence (e.g., SEQ ID NO:5); (b) a DNA sequence degenerate to a naturally occurring CD2 DNA sequence; or (c) a DNA sequence that hybridizes to one of the foregoing DNA sequences under conditions equivalent to about 20°C to 27°C below Tₘ and 1 M sodium chloride.

As used herein, "LFA-3" means an LFA-3 polypeptide that binds to a naturally occurring CD2 polypeptide and which is encoded by (a) a naturally occurring mammalian LFA-3 DNA sequence (e.g., SEQ ID NO:1 or SEQ ID NO:3); (b) a DNA sequence degenerate to a naturally occurring LFA-3 DNA sequence; or (c) a DNA sequence that hybridizes to one of the foregoing DNA sequences under conditions to about 20°C to 27°C below Tₘ and 1 M sodium chloride.

As used herein, a "soluble LFA-3 polypeptide" or a "soluble CD2 polypeptide" is an LFA-3 or CD2 polypeptide incapable of anchoring itself in a membrane. Such soluble polypeptides include, for example, CD2 and LFA-3 polypeptides that lack a sufficient portion of their membrane spanning domain to anchor the polypeptide or are modified such that the membrane spanning domain is non-functional. As used herein soluble LFA-3 polypeptides include full-length or truncated (e.g., with internal deletions) PI-linked LFA-3.

As used herein, an "antibody homolog" is a protein comprising one or more polypeptides selected from immunoglobulin light chains, immunoglobulin heavy chains and antigen-binding fragments thereof which are capable of binding to one or more antigens. The component polypeptides of an antibody homolog composed of more than one polypeptide may optionally be disulfide-bound or otherwise covalently crosslinked. Accordingly, antibody homologs include intact immunoglobulins of types IgA, IgG, IgE, IgD, IgM (as well as subtypes thereof), wherein the light chains of the immunoglobulin may be of types kappa or lambda. Antibody homologs also include portions of intact immunoglobulins that retain antigen-binding specificity, for example, Fab fragments, Fab' fragments, F(ab')₂ fragments, F(v) fragments, heavy chain monomers or dimers, light chain monomers or dimers, dimers consisting of one heavy and one light chain, and the like.

As used herein, a "humanized recombinant antibody homolog" is an antibody homolog, produced by recombinant DNA technology, in which some or all of the amino acids of a human immunoglobulin light or heavy chain that are not required for antigen binding have been substituted for the corresponding amino acids from a nonhuman mammalian immunoglobulin light or heavy chain.

As used herein, a "chimeric recombinant antibody homolog" is an antibody homolog, produced by recombinant DNA technology, in which all or part of the hinge and constant regions of an immunoglobulin light chain, heavy chain, or both, have been substituted for the corresponding regions from another immunoglobulin light chain or heavy chain.

### Skin Conditions

The methods of this invention are useful to prevent or treat mammalian, including human, skin conditions characterized by increased T cell activation and abnormal antigen presentation in the dermis and epidermis, by administering inhibitors of the CD2/LFA-3 interaction. Such conditions include psoriasis, UV damage, atopic dermatitis, cutaneous T cell lymphoma such as mycosis fungoides, allergic and irritant contact dermatitis, lichen planus, alopecia areata, pyoderma gangrenosum, vitiligo, ocular cicatricial pemphigoid, and urticaria. It is to be understood that methods of treatment and prophylaxis of skin conditions such as pyoderma gangrenosum and urticaria are included within the scope of the present invention. These latter skin conditions are also cyclosporin A sensitive dermatoses and therefore involve T cell activation. Preferably, the methods of the invention are used in the prophylaxis or treatment of psoriasis or UV damage. The methods of the invention may be practiced on any mammal, preferably on humans.

While not wishing to be bound by theory, applicants believe that inhibitors of the CD2/LFA-3 interaction used in accordance with the methods of this invention are prophylactic and therapeutic for the treatment of the aforementioned skin conditions because they inhibit the interaction between T cells and antigen presenting cells, resulting in, among other things, an inhibition of T cell proliferation and activation. Applicants believe that adverse effects of skin conditions of the type discussed herein are due to such T cell proliferation and activation. Applicants believe that the methods of the present invention are superior to previously available therapies for these skin conditions for a number of reasons, including, inhibition of antigen specific interactions for all antigens present, inhibition of T cell activation without depletion of T cells, no general immunosuppression and, possibly, induction of tolerance.

In particular, applicants believe that use of the methods of this invention will result in more specific targeting of therapy to T cells actually in the initiating stage of the lesion with no effect on polymorphonuclear leukocytes or macrophage mediated effector mechanisms. Accordingly, the patient will be less susceptible to infections than with steroids or other general immunosuppressants. Thus, methods of inhibiting T cell activation, as provided herein, are prophylactic and therapeutic for such skin conditions.

### Inhibitors Of The CD2/LFA-3 Interaction

Any inhibitor of the CD2/LFA-3 interaction is useful in the methods of this invention. Such inhibitors include anti-LFA-3 antibody homologs, anti-CD2 antibody homologs, soluble LFA-3 polypeptides, soluble CD2 polypeptides, LFA-3 and CD2 mimetic agents and derivatives thereof. Preferred inhibitors are soluble LFA-3 polypeptides and anti-LFA-3 antibody homologs.

The utility in the methods of this invention of specific soluble CD2 polypeptides, soluble LFA-3 polypeptides, anti-LFA-3 antibody homologs, anti-CD2 antibody homologs or CD2 and LFA-3 mimetic agents may easily be determined by assaying their ability to inhibit the LFA-3/CD2 interaction. This ability may be assayed, for example, using a simple cell binding assay that permits visual (under magnification) evaluation of the ability of the putative inhibitor to inhibit the interaction between LFA-3 and CD2 on cells bearing these molecules. Jurkat cells are preferred as the CD2⁺ substrate and sheep red blood cells or human JY cells are preferred as the LFA-3⁺ substrate. The binding characteristics of soluble polypeptides, antibody homologs and mimetic agents useful in this invention may be assayed in several known ways, such as by radiolabeling the antibody homolog, polypeptide or agent (e.g., ³⁵S or ¹²⁵I) and then contacting the labeled polypeptide, mimetic agent or antibody homolog with CD2⁺ of LFA-3⁺ cells, as appropriate. Binding characteristics may also be assayed using an appropriate enzymatically labelled secondary antibody. Rosetting competition assays such as those described by Seed et al. (Proc. Natl. Acad. Sci. USA, 84, pp. 3365-69 (1987)) may also be used.

### A. Anti-LFA-3 And Anti-CD2 Antibody Homologs

Many types of anti-LFA-3 or anti-CD2 antibody homologs are useful in the methods of this invention. These include monoclonal antibodies, recombinant antibodies, chimeric recombinant antibodies, humanized recombinant antibodies, as well as antigen-binding portions of the foregoing.

Among the anti-LFA-3 antibody homologs, it is preferable to use monoclonal anti-LFA-3 antibodies. It is more preferable to use a monoclonal anti-LFA-3 antibody produced by a hybridoma selected from the group of hybridomas having accession numbers ATCC HB 10693 (1E6), ATCC HB 10694 (HC-1B11), ATCC HB 10695 (7A6), and ATCC HB 10696 (8B8), or the monoclonal antibody known as TS2/9 (Sanchez-Madrid et al., "Three Distinct Antigens Associated with Human T-Lymphocyte-Mediated Cytolysis: LFA-1, LFA-2 and LFA-3", Proc. Natl. Acad. Sci. USA, 79, pp. 7489-93 (1982)). Most preferably, the monoclonal anti-LFA-3 antibody is produced by a hybridoma selected from the group of hybridomas having accession numbers ATCC HB 10695 (7A6) and ATCC HB 10693 (1E6).

Among the anti-CD2 antibody homologs, it is preferable to use monoclonal anti-CD2 antibodies, such as the anti-CD2 monoclonal antibodies known as the T11₁ epitope antibodies, including TS2/18 (Sanchez-Madrid et al., "Three Distinct Antigens Associated with Human T-Lymphocyte-Mediated Cytolysis: LFA-1, LFA-2 and LFA-3", Proc. Natl. Acad. Sci. USA, 79, pp. 7489-93 (1982)).

The technology for producing monoclonal antibodies is well known. Briefly, an immortal cell line (typically myeloma cells) is fused to lymphocytes (typically splenocytes) from a mammal immunized with preparation comprising a given antigen, and the culture supernatants of the resulting hybridoma cells are screened for antibodies against the antigen. See generally, Kohler et al., Nature, "Continuous Cultures of Fused Cells Secreting Antibody of Predefined Specificity", 256, pp. 495-97 (1975). Useful immunogens for the purpose of this invention include CD2- or LFA-3-bearing cells, as well as cell free preparations containing LFA-3, CD2 or counter receptor-binding fragments thereof (e.g., CD2 fragments that bind to LFA-3 or LFA-3 fragments that bind to CD2).

Immunization may be accomplished using standard procedures. The unit dose and immunization regimen depend on the species of mammal immunized, its immune status, the body weight of the mammal, etc. Typically, the immunized mammals are bled and the serum from each blood sample is assayed for particular antibodies using appropriate screening assays. For example, useful anti-LFA-3 or anti-CD2 antibodies may be identified by testing the ability of the immune serum to block sheep red blood cell rosetting of Jurkat cells, which results from the presence of LFA-3 and CD2 on the respective surfaces of these cells. The lymphocytes used in the production of hybridoma cells typically are isolated from immunized mammals whose sera have already tested positive for the presence of the desired antibodies using such screening assays.

Typically, the immortal cell line (e.g., a myeloma cell line) is derived from the same mammalian species as the lymphocytes. Preferred immortal cell lines are mouse myeloma cell lines that are sensitive to culture medium containing hypoxanthine, aminopterin and thymidine ("HAT medium").

Typically, HAT-sensitive mouse myeloma cells are fused to mouse splenocytes using polyethylene glycol ("PEG") 3350. Hybridoma cells resulting from the fusion are then selected using HAT medium, which kills unfused and unproductively fused myeloma cells (unfused splenocytes die after several days because they are not transformed). Hybridomas producing a desired antibody are detected by screening the hybridoma culture supernatants, for example, for the ability to bind to their respective counter receptor, or for their ability to block Jurkat cell adhesion to sheep red blood cells. Subcloning of the hybridoma cultures by limiting dilution is typically performed to ensure monoclonality.

To produce anti-LFA-3 or anti-CD2 monoclonal antibodies, hybridoma cells that tested positive in such screening assays are cultured in a nutrient medium under conditions and for a time sufficient to allow the hybridoma cells to secrete the monoclonal antibodies into the culture medium. Tissue culture techniques and culture media suitable for hybridoma cells are well known. The conditioned hybridoma culture supernatant may be collected and the desired antibodies optionally further purified by well-known methods.

Alternatively, the desired antibody may be produced by injecting the hybridoma cells into the peritoneal cavity of a pristane-primed mouse. The hybridoma cells proliferate in the peritoneal cavity, secreting the antibody, which accumulates as ascites fluid. The antibody may be harvested by withdrawing the ascites fluid from the peritoneal cavity with a syringe.

Anti-CD2 and anti-LFA-3 antibody homologs useful in the present invention may also be recombinant antibodies produced by host cells transformed with DNA encoding immunoglobulin light and heavy chains of a desired antibody. Recombinant antibodies may be produced by well known genetic engineering techniques. See, e.g., United States patent 4,816,397, which is incorporated herein by reference.

For example, recombinant antibodies may be produced by cloning cDNA or genomic DNA encoding the immunoglobulin light and heavy chains of the desired antibody from a hybridoma cell that produces an antibody homolog useful in this invention. The cDNA or genomic DNA encoding those polypeptides is then inserted into expression vectors so that both genes are operatively linked to their own transcriptional and translational expression control sequences. The expression vector and expression control sequences are chosen to be compatible with the expression host cell used. Typically, both genes are inserted into the same expression vector.

Prokaryotic or eukaryotic host cells may be used. Expression in eukaryotic host cells is preferred because such cells are more likely than prokaryotic cells to assemble and secrete a properly folded and immunologically active antibody. However, any antibody produced that is inactive due to improper folding may be renaturable according to well known methods (Kim and Baldwin, "Specific Intermediates in the Folding Reactions of Small Proteins and the Mechanism of Protein Folding", Ann. Rev. Biochem., 51, pp. 459-89 (1982)). It is possible that the host cells will produce portions of intact antibodies, such as light chain dimers or heavy chain dimers, which also are antibody homologs according to the present invention.

It will be understood that variations on the above procedure are useful in the present invention. For example, it may be desired to transform a host cell with DNA encoding either the light chain or the heavy chain (but not both) of an antibody homolog. Recombinant DNA technology may also be used to remove some or all of the DNA encoding either or both of the light and heavy chains that is not necessary for CD2 or LFA-3 counter receptor binding. The molecules expressed from such truncated DNA molecules are useful in the methods of this invention. In addition, bifunctional antibodies may be produced in which one heavy and one light chain are anti-CD2 or anti-LFA-3 antibody homologs and the other heavy and light chain are specific for an antigen other than CD2 or LFA-3, or another epitope of CD2 or LFA-3.

Chimeric recombinant anti-LFA-3 or anti-CD2 antibody homologs may be produced by transforming a host cell with a suitable expression vector comprising DNA encoding the desired immunoglobulin light and heavy chains in which all or some of the DNA encoding the hinge and constant regions of the heavy and/or the light chain have been substituted with DNA from the corresponding region of an immunoglobulin light or heavy chain of a different species. When the original recombinant antibody is nonhuman, and the inhibitor is to be administered to a human, substitution of corresponding human sequences is preferred. An exemplary chimeric recombinant antibody has mouse variable regions and human hinge and constant regions. See generally, United States patent 4,816,397 and Morrison et al., "Chimeric Human Antibody Molecules: Mouse Antigen-Binding Domains With Human Constant Region Domains", Proc. Natl. Acad. Sci. USA, 81, pp. 6851-55 (1984).

Humanized recombinant anti-LFA-3 or anti-CD2 antibodies may be produced by transforming a host cell with a suitable expression vector comprising DNA encoding the desired nonhuman immunoglobulin light and heavy chains in which all or some of the DNA encoding amino acids not involved in antigen binding have been substituted with DNA from the corresponding region of a desired human immunoglobulin light or heavy chain. See generally, Jones et al., "Replacing the Complementarity-Determining Regions in a Human Antibody with Those from a Mouse", Nature, 321, pp. 522-25 (1986).

Anti-CD2 and anti-LFA-3 antibody homologs that are not intact antibodies are also useful in this invention. Such homologs may be derived from any of the antibody homologs described above. For example, antigen-binding fragments, as well as full-length monomeric, dimeric or trimeric polypeptides derived from the above-described antibodies are themselves useful. Useful antibody homologs of this type include Fab fragments, Fab' fragments, F(ab')₂ fragments, F(v) fragments, heavy chain monomers or dimers, light chain monomers or dimers, dimers consisting of one heavy and one light chain, and the like. Anti-LFA-3 heavy chains are preferred anti-LFA-3 antibody fragments.

Antibody fragments may also be produced by chemical methods, e.g., by cleaving an intact antibody with a protease, such as pepsin or papain, and optionally treating the cleaved product with a reducing agent. Alternatively, useful fragments may be produced by using host cells transformed with truncated heavy and/or light chain genes. Heavy and light chain monomers may be produced by treating an intact antibody with a reducing agent, such as dithiothreitol, followed by purification to separate the chains. Heavy and light chain monomers may also be produced by host cells transformed with DNA encoding either the desired heavy chain or light chain, but not both. See, e.g., Ward et al., "Binding Activities of a Repertoire of Single Immunoglobulin Variable Domains Secreted from Escherichia coli", Nature, 341, pp. 544-46 (1989); Sastry et al., "Cloning of the Immunological Repertoire in Escherichia coli for Generation of Monoclonal Catalytic Antibodies: Construction of a Heavy Chain Variable Region-Specific cDNA Library", Proc. Natl. Acad. Sci. USA, 86, pp. 5728-32 (1989).

### B. Soluble CD2 and LFA-3 Polypeptides

Soluble LFA-3 polypeptides or soluble CD2 polypeptides that inhibit the interaction of LFA-3 and CD2 are useful in the methods of the present invention. Soluble LFA-3 polypeptides are preferred.

Soluble LFA-3 polypeptides may be derived from the transmembrane form of LFA-3, particularly the extracellular domain (e.g., AA₁-AA₁₈₇ of SEQ ID NO:2). Such polypeptides are described in United States patent 4,956,281 and co-pending United States patent application 07/667,971 (which shares a common assignee with the present application and which was published as PCT WO 921001. Preferred soluble LFA-3 polypeptides include polypeptides consisting of AA₁-AA₉₂ of SEQ ID No:2, AA₁-AA₈₀ of SEQ ID NO:2, AA₅₀-AA₆₅ of SEQ ID NO:2 and AA₂₀-AA₈₀ of SEQ ID NO: 2. A vector comprising a DNA sequence encoding SEQ ID NO:2 (i.e., SEQ ID No:1) is deposited with the American Type Culture Collection, Rockville, MD under accession number 75107.

Soluble LFA-3 polypeptides may also be derived from the PI-linked form of LFA-3, such as those described in PCT patent application WO 90/02181. A vector comprising a DNA sequence encoding PI-linked LFA-3 (i,e., SEQ ID NO:3) is deposited with the American Type Culture Collection, Rockville, MD under accession number 68788. It is to be understood that the PI-linked form of LFA-3 and the transmembrane form of LFA-3 have identical amino acid sequences through the entire extracellular domain. Accordingly, the preferred PI-linked LFA-3 polypeptides are the same as for the transmembrane form of LFA-3.

Soluble CD2 polypeptides may be derived from full length CD2, particularly the extracellular domain (e.g., AA₁-AA₁₈₅ of SEQ ID NO:6). Such polypeptides may comprise all or part of the extracellular domain of CD2. Exemplary soluble CD2 polypeptides are described in PCT WO 90/08187.

The production of the soluble polypeptides useful in this invention may be achieved by a variety of methods known in the art. For example, the polypeptides may be derived from intact transmembrane LFA-3 or CD2 molecules or an intact PI-linked LFA-3 molecule by proteolysis using specific endopeptidases in combination with exopeptidases, Edman degradation, or both. The intact LFA-3 molecule or the intact CD2 molecule, in turn, may be purified from its natural source using conventional methods. Alternatively, the intact LFA-3 or CD2 may be produced by known recombinant DNA techniques using cDNAs (see, e.g., U.S. Patent 4,956,281 to Wallner et al.; Aruffo and Seed, Proc. Natl. Acad. Sci., 84, pp. 2941-45 (1987); Sayre et al., Proc. Natl. Acad. Sci. USA, 84, pp. 2941-45 (1987)).

Preferably, the soluble polypeptides useful in the present invention are produced directly, thus eliminating the need for an entire LFA-3 molecule or an entire CD2 molecule as a starting material. This may be achieved by conventional chemical synthesis techniques or by well-known recombinant DNA techniques wherein only those DNA sequences which encode the desired peptides are expressed in transformed hosts. For example, a gene which encodes the desired soluble LFA-3 polypeptide or soluble CD2 polypeptide may be synthesized by chemical means using an oligonucleotide synthesizer. Such oligonucleotides are designed based on the amino acid sequence of the desired soluble LFA-3 polypeptide or soluble CD2 polypeptide. Specific DNA sequences coding for the desired peptide also can be derived from the full length DNA sequence by isolation of specific restriction endonuclease fragments or by PCR synthesis of the specified region.

Standard methods may be applied to synthesize a gene encoding a soluble LFA-3 polypeptide or a soluble CD2 polypeptide that is useful in this invention. For example, the complete amino acid sequence may be used to construct a back-translated gene. A DNA oligomer containing a nucleotide sequence coding for a soluble LFA-3 polypeptide or a soluble CD2 polypeptide useful in this invention may be synthesized in a single step. Alternatively, several smaller oligonucleotides coding for portions of the desired polypeptide may be synthesized and then ligated. Preferably, a soluble LFA-3 polypeptide or a soluble CD2 polypeptide useful in this invention will be synthesized as several separate oligonucleotides which are subsequently linked together. The individual oligonucleotides typically contain 5' or 3' overhangs for complementary assembly.

Once assembled, preferred genes will be characterized by sequences that are recognized by restriction endonucleases (including unique restriction sites for direct assembly into a cloning or an expression vector), preferred codons taking into consideration the host expression system to be used, and a sequence which, when transcribed, produces a stable, efficiently translated mRNA. Proper assembly may be confirmed by nucleotide sequencing, restriction mapping, and expression of a biologically active polypeptide in a suitable host.

It will be appreciated by those of skill in the art that, due to the degeneracy of the genetic code, DNA molecules comprising many other nucleotide sequences will also be capable of encoding the soluble LFA-3 and CD2 polypeptides encoded by the specific DNA sequences described above. These degenerate sequences also code for polypeptides that are useful in this invention.

The DNA sequences may be expressed in unicellular hosts. As is well known in the art, in order to obtain high expression levels of a transfected gene in a host, the gene must be operatively linked to transcriptional and translational expression control sequences that are functional in the chosen expression host. Preferably, the expression control sequences, and the gene of interest, will be contained in an expression vector that further comprises a bacterial selection marker and origin of replication. If the expression host is a eukaryotic cell, the expression vector should further comprise an additional expression marker useful in the expression host.

The DNA sequences encoding the desired soluble polypeptides may or may not encode a signal sequence. If the expression host is prokaryotic, it generally is preferred that the DNA sequence not encode a signal sequence. If the expression host is eukaryotic, it generally is preferred that a signal sequence be encoded.

An amino terminal methionine may or may not be present on the expressed product. If the terminal methionine is not cleaved by the expression host, it may, if desired, be chemically removed by standard techniques.

A wide variety of expression host/vector combinations may be employed. Useful expression vectors for eukaryotic hosts, include, for example, vectors comprising expression control sequences from SV40, bovine papilloma virus, adenovirus and cytomegalovirus. Useful expression vectors for bacterial hosts include known bacterial plasmids, such as plasmids from E.coli, including col E1, pCR1, pBR322, pMB9 and their derivatives, wider host range plasmids, such as RP4, phage DNAs, e.g., the numerous derivatives of phage lambda, e.g., NM989, and other DNA phages, such as M13 and filamentous single stranded DNA phages. Useful expression vectors for yeast cells include the 2µ plasmid and derivatives thereof. Useful vectors for insect cells include pVL 941.

In addition, any of a wide variety of expression control sequences may be used in these vectors. Such useful expression control sequences include the expression control sequences associated with structural genes of the foregoing expression vectors. Examples of useful expression control sequences include, for example, the early and late promoters of SV40 or adenovirus, the lac system, the trp system, the TAC or TRC system, the major operator and promoter regions of phage lambda, the control regions of fd coat protein, the promoter for 3-phosphoglycerate kinase or other glycolytic enzymes, the promoters of acid phosphatase, e.g., Pho5, the promoters of the yeast α-mating system and other sequences known to control the expression of genes of prokaryotic or eukaryotic cells or their viruses, and various combinations thereof.

A wide variety of unicellular host cells are useful. These hosts may include well known eukaryotic and prokaryotic hosts, such as strains of E.coli, Pseudomonas, Bacillus, Streptomyces, fungi, yeast, insect cells such as Spodoptera frugiperda (SF9), animal cells such as CHO and mouse cells, African green monkey cells such as COS 1, COS 7, BSC 1, BSC 40, and BMT 10, and human cells, as well as plant cells in tissue culture. For animal cell expression, we prefer CHO cells and COS 7 cells.

It should of course be understood that not all vectors and expression control sequences will function equally well to express the DNA sequences described herein. Neither will all hosts function equally well with the same expression system. However, one of skill in the art may make a selection among these vectors, expression control sequences and hosts without undue experimentation. For example, in selecting a vector, the host must be considered because the vector must replicate in it. The vector's copy number, the ability to control that copy number, and the expression of any other proteins encoded by the vector, such as antibiotic markers, should also be considered.

In selecting an expression control sequence, a variety of factors should also be considered. These include, for example, the relative strength of the sequence, its controllability, and its compatibility with the DNA sequences discussed herein, particularly as regards potential secondary structures. Unicellular hosts should be selected by consideration of their compatibility with the chosen vector, the toxicity of the product coded for by the DNA sequences, their secretion characteristics, their ability to fold the soluble polypeptides correctly, their fermentation or culture requirements, and the ease of purification of the products coded for by the DNA sequences.

Within these parameters, one of skill in the art may select various vector/expression control sequence/host combinations that will express the desired DNA sequences on fermentation or in large scale animal culture, for example with CHO cells or COS 7 cells.

The soluble LFA-3 and CD2 polypeptides may be isolated from the fermentation or cell culture and purified using any of a variety of conventional methods. One of skill in the art may select the most appropriate isolation and purification techniques.

While recombinant DNA techniques are the preferred method of producing useful soluble CD2 polypeptides or soluble LFA-3 polypeptides having a sequence of more than 20 amino acids, shorter CD2 or LFA-3 polypeptides having less than about 20 amino acids are preferably produced by conventional chemical synthesis techniques. Synthetically produced polypeptides useful in this invention can advantageously be produced in extremely high yields and can be easily purified.

Preferably, such soluble CD2 polypeptides or soluble LFA-3 polypeptides are synthesized by solution phase or solid phase polypeptide synthesis and, optionally, digested with carboxypeptidase (to remove C-terminal amino acids) or degraded by manual Edman degradation (to remove N-terminal amino acids). Proper folding of the polypeptides may be achieved under oxidative conditions which favor disulfide bridge formation as described by Kent, "Chemical Synthesis of Polypeptides and Proteins", Ann. Rev. Biochem., 57, pp. 957-89 (1988). Polypeptides produced in this way may then be purified by separation techniques widely known in the art, preferably utilizing reverse phase HPLC. The use of solution phase synthesis advantageously allows for the direct addition of certain derivatized amino acids to the growing polypeptide chain, such as the O-sulfate ester of tyrosine. This obviates the need for a subsequent derivatization step to modify any residue of the polypeptides useful in this invention.

### C. LFA-3 And CD2 Mimetic Agents

Also useful in the methods of this invention are LFA-3 and CD2 mimetic agents. These agents which may be peptides, semi-peptidic compounds or non-peptidic compounds, are inhibitors of the CD2/LFA-3 interaction. The most preferred CD2 and LFA-3 mimetic agents will inhibit the CD2/LFA-3 interaction at least as well as anti-LFA-3 monoclonal antibody 7A6 or anti-CD2 monoclonal antibody TS2/18 (described supra).

Such mimetic agents may be produced by synthesizing a plurality of peptides (e.g., 5-20 amino acids in length), semi-peptidic compounds or non-peptidic, organic compounds, and then screening those compounds for their ability to inhibit the CD2/LFA-3 interaction. See generally United States patent 4,833,092, Scott and Smith, "Searching for Peptide Ligands with an Epitope Library", Science, 249, pp. 386-90 (1990), and Devlin et al., "Random Peptide Libraries: A Source of Specific Protein Binding Molecules", Science, 249, pp. 404-07 (1990), which are herein incorporated by reference.

### D. Derivatized Inhibitors

Also useful in the methods of this invention are derivatized inhibitors of the CD2/LFA-3 interaction in which, for example, any of the antibody homologs, soluble CD2 and LFA-3 polypeptides, or CD2 and LFA-3 mimetic agents described herein are functionally linked (by chemical coupling, genetic fusion or otherwise) to one or more members independently selected from the group consisting of anti-LFA-3 and anti-CD2 antibody homologs, soluble LFA-3 and CD2 polypeptides, CD2 and LFA-3 mimetic agents, cytotoxic agents and pharmaceutical agents.

One type of derivatized inhibitor is produced by crosslinking two or more inhibitors (of the same type or of different types). Suitable crosslinkers include those that are heterobifunctional, having two distinctly reactive groups separated by an appropriate spacer (e.g., m-maleimidobenzoyl-N-hydroxysuccinimide ester) or homobifunctional (e.g., disuccinimidyl suberate). Such linkers are available from Pierce Chemical Company, Rockford, IL.

Another possibility for cross-linking takes advantage of the PI linkage signal sequence in PI-linked LFA-3, or fragments thereof. Specifically, DNA encoding the PI-linkage signal sequence (e.g., AA₁₆₂-AA₂₁₂ of SEQ ID NO:4) is ligated downstream of DNA encoding a desired polypeptide, preferably a soluble LFA-3 polypeptide. If this construct is expressed in an appropriate eukaryotic cell, the cell will recognize the PI linkage signal sequence and will covalently link PI to the polypeptide. The hydrophobic property of the PI may then be exploited to form micellar aggregates of the polypeptides.

Also useful are inhibitors linked to one or more cytotoxic or pharmaceutical agents. Useful pharmaceutical agents include biologically active peptides, polypeptides and proteins, such as antibody homologs specific for a human polypeptide other than CD2 or LFA-3, or portions thereof. Useful pharmaceutical agents and cytotoxic agents also include cyclosporin A, prednisone, FK506, methotrexate, steroids, retinoids, interferon, and nitrogen mustard.

Preferred inhibitors derivatized with a pharmaceutical agent include recombinantly-produced polypeptides in which a soluble LFA-3 polypeptide, soluble CD2 polypeptide, or a peptidyl CD2 or peptidyl LFA-3 mimetic agent is fused to all or part of an immunoglobulin heavy chain hinge region and all or part of a heavy chain constant region. Preferred polypeptides for preparing such fusion proteins are soluble LFA-3 polypeptides. Most preferred are fusion proteins containing AA₁-AA₉₂ of LFA-3 (e.g., SEQ ID NO:2) fused to a portion of a human IgG₁ hinge region (including the C-terminal ten amino acids of the hinge region containing two cysteine residues thought to participate in inter-chain disulfide bonding) and the CH2 and CH3 regions of an IgG₁ heavy chain constant domain. Such fusion proteins are expected to exhibit prolonged serum half-lives and enable inhibitor dimerization.

### Pharmaceutical Compositions And Methods According To This Invention

This invention provides a method for preventing or treating the above-mentioned skin conditions in a mammal by administering to the mammal one or more inhibitors of the CD2/LFA-3 interaction, or derivatized form(s) thereof.

Preferably, an effective amount of the inhibitor or derivatized form thereof is administered. By "effective amount" is meant an amount capable of lessening the spread or severity of the skin conditions described herein.

It will be apparent to those of skill in the art that the effective amount of inhibitor will depend, inter alia, upon the administration schedule, the unit dose administered, whether the inhibitor is administered in combination with other therapeutic agents, the immune status and health of the patient, the therapeutic or prophylactic activity of the particular inhibitor administered and the serum half-life.

Preferably, the inhibitor is administered at a dose between about 0.001 and about 50 mg inhibitor per kg body weight, more preferably, between about 0.01 and about 10 mg inhibitor per kg body weight, most preferably between about 0.1 and about 4 mg inhibitor per kg body weight.

Unit doses should be administered until an effect is observed. The effect may be measured by a variety of methods, including, in vitro T cell activity assays and clearing of affected skin areas. Preferably, the unit dose is administered about one to three times per week or one to three times per day. More preferably, it is administered about one to three times per day for between about 3 and 7 days, or about one to three times per day for between about 3 and 7 days on a monthly basis. It will be recognized, however, that lower or higher dosages and other administrations schedules may be employed.

The inhibitor(s) or derivatized form(s) thereof are also preferably administered in a composition including a pharmaceutically acceptable carrier. By "pharmaceutically acceptable carrier" is meant a carrier that does not cause an allergic reaction or other untoward effect in patients to whom it is administered.

Suitable pharmaceutically acceptable carriers include, for example, one or more of water, saline, phosphate buffered saline, dextrose, glycerol, ethanol and the like, as well as combinations thereof. Pharmaceutically acceptable carriers may further comprise minor amounts of auxiliary substances such as wetting or emulsifying agents, preservatives or buffers, which enhance the shelf life or effectiveness of the inhibitor.

The pharmaceutical composition or inhibitor may be administered in conjunction with other therapeutic or prophylactic agents. These include, for example, cyclosporin A, steroids, retinoids, nitrogen mustard, interferon, methotrexate, antibiotics and antihistamines.

These agents may be administered in single dosage form with the inhibitor (i.e., as part of the same pharmaceutical composition), a multiple dosage form separately from the inhibitor, but concurrently, or a multiple dosage form wherein the two components are administered separately but sequentially. Alternatively, the inhibitor and the other active agent may be in the form of a single conjugated molecule. Conjugation of the two components may be achieved by standard cross-linking techniques well known in the art. A single molecule may also take the form of a recombinant fusion protein. In addition, the inhibitors, or pharmaceutical compositions, useful in the present invention may be used in combination with other therapies such as PUVA, chemotherapy and UV light. Such combination therapies may advantageously utilize lower dosages of the therapeutic or prophylactic agents.

The inhibitor, or pharmaceutical composition, may be in a variety of forms. These include, for example, solid, semi-solid and liquid dosage forms, such as tablets, pills, powders, liquid solutions, dispersions or suspensions, liposomes, suppositories, injectable infusible, and topical preparations. The preferred form depends on the intended mode of administration and therapeutic application. The preferred forms are injectable or infusible solutions.

The inhibitor or pharmaceutical composition may be administered intravenously, intramuscularly, subcutaneously, intra-articularly, intrathecally, periostally, intratumorally, intralesionally, perilesionally by infusion, orally, topically or by inhalation. Preferably it is administered subcutaneously, intramuscularly or intravenously. Most preferably, it is administered subcutaneously.

In order that this invention may be better understood, the following examples are set forth. These examples are for purposes of illustration only, and are not to be construed as limiting the scope of the invention in any manner.

### EXAMPLE 1

### Subjects

Six adult patients participated in the investigation. Informed consent was obtained after Internal Review Board approval of the protocol. All patients satisfied the major diagnostic criteria for psoriasis, namely chronic papulosquamous plaques of characteristic morphology and distribution. The intermittent use of topical corticosteroids was common among these patients but was discontinued 2 weeks prior to entry into the study. A group of healthy volunteers with no history of psoriasis or other skin disease was utilized as the normal control group.

### Preparation of Epidermal Cell Suspensions

Skin biopsy specimens were obtained from both normal and lesional skin by using a keratome. The specimens were submerged in Dulbecco's phosphate buffered saline ("PBS") (Gibco Labs, Grand Island, NY) containing 50 units/ml dispase (Collaborative Research, Bedford, MA). The specimens were then incubated at 4°C for 18 hours and the epidermis removed from the remaining dermis.

Epidermal sheets were removed from the dermis, submerged in Dulbecco's PBS containing 0.5% trypsin (Sigma Chemical Co., St. Louis, MO), and incubated at 37°C for 30 minutes.

Trypsinized epidermal sheets were transferred to 0.05% DNase (Sigma) in Dulbecco's PBS where they were teased into a cell suspension. Fetal bovine serum ("FBS") (Hyclone, Logan, UT) was added to inactivate residual trypsin and the epidermal cell suspension then passed through a 112 µm nylon filter (Tetko, Elmsford, NY). After washing the predominantly single cell suspension three times in Dulbecco's PBS with 1% FBS, cells were resuspended in culture media which consisted of RPMI 1640 (Whittaker MA Bioproducts, Wakerfield, MD) containing 1% penicillin and streptomycin, 1% glutamine (Gibco), and 10% human AB serum (Sigma).

### Isolation and Depletion of T cells

Peripheral blood mononuclear cells ("MNC") were isolated from heparinized blood using Ficoll Hypaque (Pharmacia) density gradient centrifugation according to manufacturer's suggested protocol. Macrophages were removed by plastic adherence at 37°C for 1 hour. The nonadherent, macrophage-depleted MNC were washed, and then depleted of CD8⁺ T lymphocytes, activated T cells, B cells, antigen presenting cells and NK cells by incubation with monoclonal antibodies to CD8 (ATCC CRL 8014), HLA-DR (ATCC CRL H355), and CD11b (ATCC CRL 8026). These antibodies were used as dilutions in PBS (1:200) of ascites fluid from pristane-primed mice.

The antibody treated MNC were incubated at 4°C with 4.5 nm magnetic particles coated with goat anti-mouse IgG (Dynabeads M-450, Dynal, Oslo, Norway) at a ratio of 3 beads per cell. Antigen positive cells were depleted by being drawn by a magnet (Advanced Magnetics, Cambridge, MA) against the side of the tube allowing the remaining cells in suspension to be decanted. The decanted cell suspension was again exposed to a magnet and cells remaining in suspension collected. Fresh goat anti-mouse IgG beads were again added to the collected cells in suspension in order to deplete any remaining antigen positive cells, and the magnetic removal process repeated. Cells were washed in PBS and resuspended in culture media prior to use. This treatment results in a preparation of resting CD4⁺ T lymphocytes enriched to 99% purity and devoid of intrinsic antigen presenting activity.

### Proliferative Response of T Lymphocytes to Autologous Psoriatic Cells

One hundred thousand CD4⁺ T lymphocytes were added to round bottom microtiter wells (Costar, Cambridge, MA) with eighty thousand psoriatic epidermal cells in 0.2 ml of RPMI containing 10% human AB serum (Sigma, St. Louis, MO). This number of psoriatic epidermal cells per well was chosen because previous experiments demonstrated that this number is sufficient to induce autoreactive T cell responses. After incubation at 37°C in 5% CO₂/95% air for 6 days, 1 µCi of [³H]TdR (ICN Radiochemicals, Irvine, CA) was added per well and the cells harvested 18 hours later on a PHD cell harvester (Cambridge Technology Inc., Cambridge, MA). The [³H]TdR incorporation was measured on a Packard scintillation counter (Packard Instrument Co., Downers Grover, IL). [³H]TdR incorporation is a measure of T cell proliferation.

Appropriate controls for T cells ("TC") alone or epidermal cells ("EC") alone were carried out using the above protocol. No [³H]TdR incorporation was observed in these assays (data not shown). Brisk proliferation of autologous T cells in response to psoriatic skin cells was observed (data not shown).

In addition, to test the allogeneic response to normal skin, the above protocol was carried out using one hundred thousand allogeneic T cells and eighty thousand normal skin cells. Under these conditions, a brisk proliferation of allogeneic T cells was observed (data not shown).

### Blocking of Psoriatic Epidermal Cells' Ability To Stimulate Autologous T Lymphocyte Proliferation

The effect on [³H]TdR incorporation (i.e., T cell proliferation) of an anti-CD2 monoclonal antibody (TS2/18) (Sanchez-Madrid et al., "Three Distinct Antigens Associated with Human T-lymphocyte-mediated Cytolysis: LFA-1, LFA-2, and LFA-3", Proc. Natl. Acad. Sci. USA, 79, pp. 7489-93 (1982)), an anti-LFA-3 monoclonal antibody (7A6) (ATCC HB 10695), or an isotype-matched, control monoclonal antibody of irrelevant specificity (MOPC21, Sigma Chemical Co., St. Louis, MO) was measured using the protocol outlined above in the presence of 50 µg/ml of the respective antibodies.

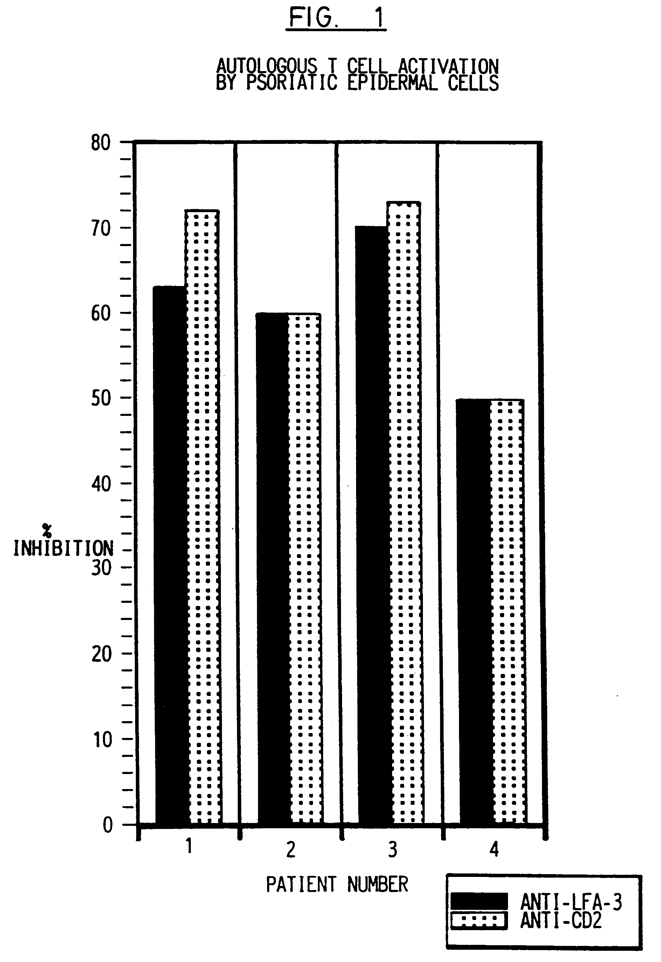

Figure 1 demonstrates that addition of anti-CD2 or anti-LFA-3 resulted in a consistent (n=4) and substantial (approximately 60%) inhibition of autologous T cell proliferation in response to lesional psoriatic epidermis, as compared to proliferation in the presence of the isotype-matched control antibody.

Figure 1 displays data for four patients only. These four patients demonstrated autoreactivity of blood CD4⁺ T cells to their own lesional epidermis, despite the fact that no antigen was added to the system. This is an abnormal finding; normal individuals' cocultures of autologous blood T cells and epidermal cells do not react. Such a reaction is considered to be an in vitro model of autoimmune reactions occurring in the skin. EC preparations from two additional patients were not informative. One EC preparation was bacterially contaminated; the other contained antigen presenting cells that did not induce autoreactive T cell responses.

Addition of 50 µg per ml of the anti-CD2 or anti-LFA-3 antibodies to the allogeneic normal skin assay described above also resulted in an inhibition of allogeneic T cell activation. The degree of inhibition was not as substantial (approximately 40%) as that observed for autologous antigen presenting cell activity when using lesional psoriatic epidermis (data not shown).

Addition of the isotype-matched control antibody (specific for an irrelevant antigen) did not significantly alter the level of T cell proliferation of autologous T cells induced by lesional psoriatic epidermis (data not shown).

### EXAMPLE 2

### Subject

One adult subject participated in this investigation. Informed consent was obtained after Internal Review Board approval of the protocol. The minimal dose of UV B from a bank of fluorescent bulbs (FS 40) required to induce skin erythema in the subject was determined prior to the study. A moderate sunburn (4 miminal erythemal doses) was then administered to the left buttock, which 3 days later was the source of UV damaged skin. Skin from the right buttock, which was unburned, was utilized for the control.

### Preparation of Epidermal Cell Suspensions

Skin biopsy specimens were obtained from both normal and sunburned skin by using a keratome. Epidermal cell suspensions were prepared from these specimens using substantially the same protocol as in Example 1.

### Isolation and Depletion of T cells

Peripheral blood mononuclear cells ("MNC") were isolated from heparinized blood of another person, using Ficoll Hypaque (Pharmacia) density gradient centrifugation according to manufacturer's suggested protocol. CD4⁺ T lymphocytes were then prepared substantially as outlined in Example 1.

### Proliferative Response Of T Lymphocytes To Allogeneic UV Damaged Epidermal Cells

One hundred thousand CD4⁺ T lymphocytes from another individual were added to round bottom microtiter wells (Costar, Cambridge, MA) with UV damaged epidermal cells from the subject, incubated in the presence of [³H]TdR, harvested and [³H]TdR incorporation was measured substantially as outlined in Example 1. This example differs from Example 1 in that the antigenic stimulus is alloantigen, rather than autoantigens that are stimulatory in psoriasis. Thus, allogeneic T cells were used, rather than autologous T cells.

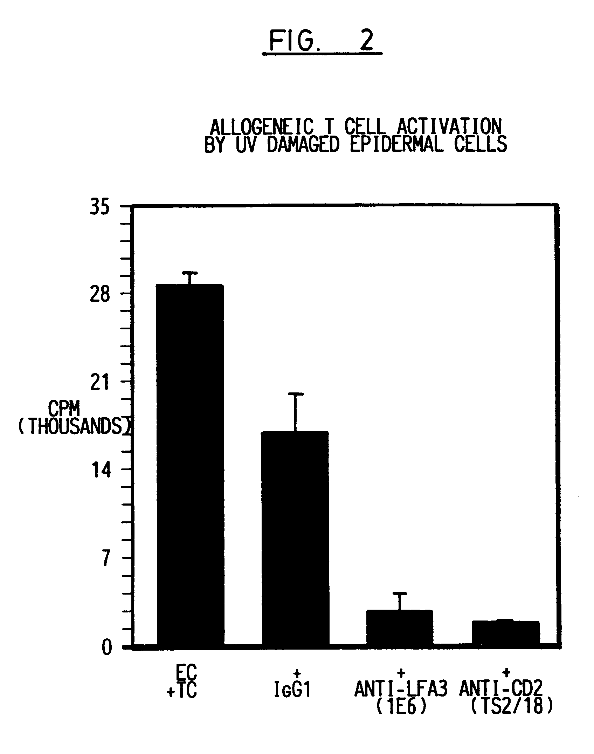

Figure 2 shows a brisk proliferation of allogeneic T cells (as measured by [³H]TdR incorporation) when incubated with UV damaged epidermal cells ("EC+TC").

### Blocking Of UV Damaged Epidermal Cells' Ability To Stimulate Allogeneic T Lymphocyte Proliferation

The effect on [³H]TdR incorporation (i.e., T cell proliferation) of an anti-LFA-3 monoclonal antibody (1E6) (ATCC HB 10693), an anti-CD2 monoclonal antibody (TS2/18) (Sanchez-Madrid et al., "Three Distinct Antigens Associated With Human T-lymphocyte-Mediated Cytolysis: LFA-1, LFA-2, and LFA-3", Proc. Natl. Acad. Sci USA, 79, pp. 7489-93 (1982)), and an isotype-matched, control monoclonal antibody of irrelevant specificity (MOPC21, Sigma Chemical Co.), was measured using the protocol outlined above in the presence of 50 µg/ml of the respective antibodies.

Figure 2 shows that in the presence of a monoclonal antibody of irrelevant specificity (MOPC21, Sigma Chemical Co.), [³H]TdR incorporation was somewhat reduced. However, the addition of anti-LFA-3 monoclonal antibody 1E6 or anti-CD2 monoclonal antibody TS2/18 resulted in a substantial inhibition of T cell proliferation compared to proliferation in the presence of the control antibody.

### Deposits

Murine hybridoma cells and anti-LFA-3 antibodies useful in the present invention are exemplified by cultures deposited under the Budapest Treaty with American Type Culture Collection, Rockville, Maryland, U.S.A., on March 5, 1991, and identified as:

| Designation | ATCC Accession No. |
|---|---|
| 1E6 | HB 10693 |
| HC-1B11 | HB 10694 |
| 7A6 | HB 10695 |
| 8B8 | HB 10696 |

A bacteriophage carrying a plasmid encoding transmembrane LFA-3 was deposited under the Budapest Treaty with In Vitro International, Inc., Linthicum, Maryland, U.S.A., on May 28, 1987 under accession number IVI-10133. This deposit was transferred to American Type Culture Collection on June 20, 1991 and identified as:

| Designation | ATCC Accession No. |
|---|---|
| λHT16[λgt10/LFA-3] | 75107 |

E. coli transformed with a plasmid encoding PI-linked LFA-3 was deposited under the Budapest Treaty with In Vitro International, Inc. on July 22, 1988 under accession number IVI-10180. This deposit was transferred to American Type Culture Collection on June 20, 1991 and identified as:

| Designation | ATCC Accession No. |
|---|---|
| p24 | 68788 |

### Sequences

The following is a summary of the sequences set forth in the Sequence Listing:
SEQ ID NO:1 DNA sequence of transmembrane LFA-3
SEQ ID NO:2 Amino acid sequence of transmembrane LFA-3
SEQ ID NO:3 DNA sequence of PI-linked LFA-3
SEQ ID NO:4 Amino acid sequence of PI-linked LFA-3
SEQ ID NO:5 DNA sequence of CD2
SEQ ID NO:6 Amino acid sequence of CD2

While we have hereinbefore described a number of embodiments of this invention, it will be appreciated that the invention is not to be limited by the specific embodiments that have been presented herein by way of example.

## Claims

1. The use of an inhibitor of the CD2/LFA-3 interaction for the manufacture of a medicament for preventing or treating human skin conditions characterized by increased T cell activation and abnormal antigen presentation in the dermis and epidermis.

2. The use as claimed in claim 1, wherein the condition is selected from the group consisting of atopic dermatitis, cutaneous T cell lymphoma such as mycosis fungoides, allergic and irritant contact dermatitis, lichen planus, alopecia areata, pyoderma gangrenosum, vitiligo, ocular cicatricial pemphigoid, and urticaria.

3. The use as claimed in claim 1, wherein the condition is psoriasis.

4. The use as claimed in claim 1, wherein the inhibitor is selected from the group consisting of anti-LFA-3 antibody homologs, anti-CD2 antibody homologs, soluble LFA-3 polypeptides and soluble CD2 polypeptides.

5. The use as claimed in claim 4, wherein the inhibitor is an anti-LFA-3 antibody homolog or an anti-CD2 antibody homolog.

6. The use as claimed in claim 5, wherein the inhibitor is a monoclonal anti-LFA-3 antibody or a monoclonal anti-CD2 antibody.

7. The use as claimed in claim 6, wherein the inhibitor is a monoclonal anti-LFA-3 antibody produced by a hybridoma selected from the group of hybridomas having accession numbers ATCC HB 10693 (1E6), ATCC HB 10694 (HC-1B11), ATCC HB 10695 (7A6), and ATCC HB 10696 (8B8) or is monoclonal antibody TS2/9.

8. The use as claimed in claim 7, wherein the monoclonal anti-LFA-3 antibody is produced by a hybridoma selected from the group of hybridomas having accession numbers ATCC HB 10695 (7A6) and ATCC HB 10693 (1E6).

9. The use as claimed in claim 5, wherein the inhibitor is a chimeric recombinant anti-LFA-3 antibody homolog or a chimeric recombinant anti-CD2 antibody homolog.

10. The use as claimed in claim 5, wherein the inhibitor is a humanized recombinant anti-LFA-3 antibody homolog or a humanized recombinant anti-CD2 antibody homolog.

11. The use as claimed in claim 5, wherein the inhibitor is selected from the group consisting of Fab fragments, Fab' fragments, F(ab')₂ fragments, F(v) fragments and intact immunoglobulin heavy chains of an anti-LFA-3 antibody homolog or an anti-CD2 antibody homolog.

12. The use as claimed in claim 4, wherein the inhibitor is a soluble CD2 polypeptide or a soluble LFA-3 polypeptide.

13. The use as claimed in claim 12, wherein the inhibitor is a soluble LFA-3 polypeptide selected from the group of polypeptides consisting of AA₁-AA₉₂ of SEQ ID NO:2, AA₁-AA₈₀ of SEQ ID NO:2, AA₅₀-AA₆₅ of SEQ ID NO:2, and AA₂₀-AA₈₀ of SEQ ID NO:2.

14. The use as claimed in claim 4, wherein the inhibitor is linked to one or more members independently selected from the group consisting of anti-LFA-3 antibody homologs, anti-CD2 antibody homologs, soluble LFA-3 polypeptides, soluble CD2 polypeptides, cytotoxic agents and pharmaceutical agents.

15. The use as claimed in claim 14, wherein the inhibitor is a polypeptide consisting of a soluble LFA-3 polypeptide linked to an immunoglobulin hinge and heavy chain constant region or portions thereof.

16. The use as claimed in claim 1, wherein the condition is UV damage.

17. An inhibitor of the CD2/LFA-3 interaction which is a polypeptide consisting of a soluble LFA-3 polypeptide linked to an immunoglobulin hinge and heavy chain constant region or portions thereof, for use in a method of therapy practised on the human or animal body.

## Patentansprüche

1. Die Verwendung eines Inhibitors der CD2/LFA-3 Wechselwirkung für die Herstellung eines Medikaments zum Vorbeugen oder Behandeln von menschlichen Hautkrankheiten, gekennzeichnet durch erhöhte T Zellen Aktivierung und abnorme Antigenpräsentation in der Dermis und Epidermis.

2. Die Verwendung nach Anspruch 1, wobei die Krankheit ausgewählt ist aus atopischer Dermatitis, Haut-T-Zellen-Lymphom wie Mycosisfungoide, Allergie- und Reizkontaktdermatitis, Lichen planus (lichen planus), Alopezie areata (alopecia areata), Pyodermie gangrenosum (pyoderma gangrenosum), Vitiligo, okulares vernarbendes Pemphigoid und Urtikaria.

3. Die Verwendung nach Anspruch 1, wobei die Krankheit Schuppenflechte ist.

4. Die Verwendung nach Anspruch 1, wobei der Inhibitor ausgewählt ist aus Anti-LFA-3 Antikörperhomologen, Anti-CD2 Antikörperhomologen, löslichen LFA-3 Polypeptiden und löslichen CD2 Polypeptiden.

5. Die Verwendung nach Anspruch 4, wobei der Inhibitor ein Anti-LFA-3 Antikörperhomolog oder ein Anti-CD2 Antikörperhomolog ist.

6. Die Verwendung nach Anspruch 5, wobei der Inhibitor ein monoklonaler Anti-LFA-3 Antikörper oder ein monoklonaler Anti-CD2 Antikörper ist.

7. Die Verwendung nach Anspruch 6, wobei der Inhibitor ein monoklonaler Anti-LFA-3 Antikörper, hergestellt mittels eines Hybridoms ausgewählt aus Hybridomen mit den Hinterlegungsnummern ATCC HB 10693 (1E6), ATCC HB 10694 (HC-1B11), ATCC HB 10695 (7A6) und ATCC HB 10696 (8B8) ist, oder monoklonaler Antikörper TS2/9 ist.

8. Die Verwendung nach Anspruch 7, wobei der monoklonale Anti-LFA-3 Antikörper mithilfe eines Hybridoms ausgewählt aus Hybridomen mit den Hinterlegungsnummern ATCC HB 10695 (7A6) und ATCC HB 10693 (1E6) hergestellt ist.

9. Die Verwendung nach Anspruch 5, wobei der Inhibitor ein rekombinantes Chimären-anti-LFA-3 Antikörperhomolog oder ein rekombinantes Chimären-anti-CD2 Antikörperhomolog ist.

10. Die Verwendung nach Anspruch 5, wobei der Inhibitor ein vermenschlichtes rekombinantes Anti-LFA-3 Antikörperhomolog oder ein vermenschlichtes rekombinantes Anti-CD2 Antikörperhomolog ist.

11. Die Verwendung nach Anspruch 5, wobei der Inhibitor aus Fab Fragmenten, Fab' Fragmenten, F(ab')₂ Fragmenten, F(v) Fragmenten und intakten schweren Immunglobulinketten eines Anti-LFA-3 Antikörperhomologen oder eines Anti-CD2 Antikörperhomologen ausgewählt ist.

12. Die Verwendung nach Anspruch 4, wobei der Inhibitor ein lösliches CD2 Polypeptid oder ein lösliches LFA-3 Polypeptid ist.

13. Die Verwendung nach Anspruch 12, wobei der Inhibitor ein lösliches LFA-3 Polypeptid ausgewählt aus Polypeptiden aus AA₁-AA₉₂ von SEQ ID NO:2, AA₁-AA₈₀ von SEQ ID NO:2, AA₅₀-AA₆₅ von SEQ ID NO:2 und AA₂₀-AA₈₀ von SEQ ID NO:2 ist.

14. Die Verwendung nach Anspruch 4, wobei der Inhibitor an ein oder mehrere Mitglieder gebunden ist, die unabhängig aus Anti-LFA-3 Antikörperhomologen, Anti-CD2 Antikörperhomologen, löslichen LFA-3 Polypeptiden, löslichen CD2 Polypeptiden, zytotoxischen Mitteln und pharmazeutischen Mitteln ausgewählt sind.

15. Die Verwendung nach Anspruch 14, wobei der Inhibitor ein Polypeptid ist, welches aus einem löslichen LFA-3 Polypeptid besteht, welches mit einem Immunglobulingelenk und konstanter schwerkettiger Region oder Teilen davon verknüpft ist.

16. Die Verwendung nach Anspruch 1, wobei die Krankheit UV Beschädigung ist.

17. Ein Inhibitor der CD2/LFA-3 Wechselwirkung, welcher ein Polypeptid ist, das aus einem löslichen LFA-3 Polypeptid besteht, das mit einem Immunglobulingelenk und konstanter schwerkettiger Region oder Teilen davon verknüpft ist, für die Verwendung in einem Therapieverfahren, welches an dem menschlichen oder tierischen Körper praktiziert wird.

## Revendications

1. Utilisation d'un inhibiteur de l'interaction entre CD2 et LFA-3 pour fabriquer un médicament destiné à prévenir ou à traiter des maladies de la peau humaines caractérisées par une activation accrue des cellules T et par une présentation anormale des antigènes dans le derme et l'épiderme.

2. Utilisation selon la revendication 1, dans laquelle la maladie est prise dans le groupe constitué de la dermatite atopique, des lymphomes de cellules T cutanées tels que le mycosis fongoïde, de l'eczéma allergique ou de l'eczéma causé par un contact avec des substances irritantes, du lichen plan, de l'alopécie en aires, de la pyoderma gangrenosum, du vitiligo, du pemphigus cicatriciel oculaire, et de l'urticaire.

3. Utilisation selon la revendication 1, dans laquelle la maladie est le psoriasis.

4. Utilisation selon la revendication 1, dans laquelle l'inhibiteur est choisi dans le groupe constitué des homologues d'anticorps anti-LFA-3, des homologues d'anticorps anti-CD2, des polypeptides LFA-3 solubles et des polypeptides CD2 solubles.

5. Utilisation selon la revendication 4, dans laquelle l'inhibiteur est un homologue d'anticorps anti-LFA-3 ou un homologue d'anticorps anti-CD2.

6. Utilisation selon la revendication 5, dans laquelle l'inhibiteur est un anticorps monoclonal anti-LFA-3 ou un anti corps monoclonal anti-CD2.

7. Utilisation selon la revendication 6, dans laquelle l'inhibiteur est un anticorps monoclonal anti-LFA-3 produit par un hybridome choisi dans le groupe constitué des hybridomes ayant les numéros d'enregistrement ATCC HB 10693 (1E6), ATCC HB 10694 (HC-1B11), ATCC HB 10695 (7A6), et ATCC HB 10696 (8B8), ou bien est l'anticorps monoclonal TS2/9.

8. Utilisation selon la revendication 7, dans laquelle l'anticorps monoclonal anti-LFA-3 est produit par un hybridome choisi dans le groupe constitué des hybridomes ayant les numéros d'enregistrement ATCC HB 10695 (7A6) et ATCC HB 10693 (1E6).

9. Utilisation selon la revendication 5, dans laquelle l'inhibiteur est un homologue d'anticorps anti-LFA-3 recombinant chimère ou un homologue d'anticorps anti-CD2 recombinant chimère.

10. Utilisation selon la revendication 5, dans laquelle l'inhibiteur est un homologue d'anticorps anti-LFA-3 recombinant humanisé ou un homologue d'anticorps anti-CD2 recombinant humanisé.

11. Utilisation selon la revendication 5, dans laquelle l'inhibiteur est choisi dans le groupe constitué des fragments Fab, des fragments Fab', des fragments F(ab')₂, des fragments F(v) et des chaînes lourdes d'immunoglobuline entières d'un homologue d'anticorps anti-LFA-3 ou d'un homologue d'anticorps anti-CD2.

12. Utilisation selon la revendication 4, dans laquelle l'inhibiteur est un polypeptide CD2 soluble ou un polypeptide LFA-3 soluble.

13. Utilisation selon la revendication 12, dans laquelle l'inhibiteur est un polypeptide LFA-3 soluble choisi dans le groupe des polypeptides constitués de la portion AA₁-AA₉₂ de la séquence ID n°2, de la portion AA₁-AA₈₀ de la séquence ID n°2, de la portion AA₅₀-AA₆₅ de la séquence ID n°2, et de la portion AA₂₀-AA₈₀ de la séquence ID n°2.

14. Utilisation selon la revendication 4, dans laquelle l'inhibiteur est lié à un ou plusieurs éléments choisis indépendamment les uns des autres dans le groupe constitué des homologues d'anticorps anti-LFA-3, des homologues d'anticorps anti-CD2, des polypeptides LFA-3 solubles, des polypeptides CD2 solubles, des agents cytotoxiques et des agents pharmaceutiques.

15. Utilisation selon la revendication 14, dans laquelle l'inhibiteur est un polypeptide constitué d'un polypeptide LFA-3 soluble lié à une région charnière d'immunoglobuline et à une région constante de chaîne lourde d'immunoglobuline, ou à des fragments de celles-ci.

16. Utilisation selon la revendication 1, dans laquelle la maladie est une lésion causée par les rayons ultraviolets.

17. Inhibiteur de l'interaction entre CD2 et LFA-3, ledit inhibiteur étant un polypeptide constitué d'un polypeptide LFA-3 soluble lié à une région charnière d'immunoglobuline et à une région constante de chaîne lourde d'immunoglobuline ou à des fragments de celles-ci, destiné à être utilisé dans une méthode de thérapie mise en oeuvre sur le corps d'un animal ou d'un être humain.


SEQUENCE LISTING

(1) GENERAL INFORMATION:

(i) APPLICANT: WALLNER, Barbara P.
COOPER, Kevin D.

(ii) TITLE OF INVENTION: METHOD OF PROPHYLAXIS OR TREATMENT OF
ANTIGEN PRESENTING CELL DRIVEN SKIN CONDITIONS USING
INHIBITORS OF THE CD2/LFA-3 INTERACTION

(iii) NUMBER OF SEQUENCES: 6

(iv) CORRESPONDENCE ADDRESS:
(A) ADDRESSEE: c\o FISH & NEAVE
(B) STREET: 875 Third Avenue
(C) CITY: New York
(D) STATE: New York
(E) COUNTRY: U.S.A.
(F) ZIP: 10022

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(viii) ATTORNEY/AGENT INFORMATION:
(A) NAME: Haley Jr., James F.
(B) REGISTRATION NUMBER: 27,794
(C) REFERENCE/DOCKET NUMBER: B167CIP

(ix) TELECOMMUNICATION INFORMATION:
(A) TELEPHONE: (212) 715-0600

(2) INFORMATION FOR SEQ ID NO:1:

(i) SEQUENCE CHARACTERISTICS:
(A) LENGTH: 753 base pairs
(B) TYPE: nucleic acid
(C) STRANDEDNESS: single
(D) TOPOLOGY: linear

(ix) FEATURE:
(A) NAME/KEY: CDS
(B) LOCATION: 1..750

(ix) FEATURE:
(A) NAME/KEY: sig_peptide
(B) LOCATION: 1..84

(ix) FEATURE:
(A) NAME/KEY: mat_peptide
(B) LOCATION: 85..750

(ix) FEATURE:
(A) NAME/KEY: misc_feature
(B) LOCATION: 1..750
(D) OTHER INFORMATION: /note= "Human transmembrane LFA-3"

(ix) FEATURE:
(A) NAME/KEY: misc_feature
(B) LOCATION: 646..714
(D) OTHER INFORMATION: /note= "Transmembrane domain"

(xi) SEQUENCE DESCRIPTION: SEQ ID NO:1:

ATG GTT GCT GGG AGC GAC GCG GGG CGG GCC CTG GGG GTC CTC AGC GTG 48
Met Val Ala Gly Ser Asp Ala Gly Arg Ala Leu Gly Val Leu Ser Val
-28 -25 -20 -15

GTC TGC CTG CTG CAC TGC TTT GGT TTC ATC AGC TGT TTT TCC CAA CAA 96
Val Cys Leu Leu His Cys Phe Gly Phe Ile Ser Cys Phe Ser Gln Gln
-10 -5 1

ATA TAT GGT GTT GTG TAT GGG AAT GTA ACT TTC CAT GTA CCA AGC AAT 144
Ile Tyr Gly Val Val Tyr Gly Asn Val Thr Phe His Val Pro Ser Asn
5 10 15 20

GTG CCT TTA AAA GAG GTC CTA TGG AAA AAA CAA AAG GAT AAA GTT GCA 192
Val Pro Leu Lys Glu Val Leu Trp Lys Lys Gln Lys Asp Lys Val Ala
25 30 35

GAA CTG GAA AAT TCT GAA TTC AGA GCT TTC TCA TCT TTT AAA AAT AGG 240
Glu Leu Glu Asn Ser Glu Phe Arg Ala Phe Ser Ser Phe Lys Asn Arg
40 45 50

GTT TAT TTA GAC ACT GTG TCA GGT AGC CTC ACT ATC TAC AAC TTA ACA 288
Val Tyr Leu Asp Thr Val Ser Gly Ser Leu Thr Ile Tyr Asn Leu Thr
55 60 65

TCA TCA GAT GAA GAT GAG TAT GAA ATG GAA TCG CCA AAT ATT ACT GAT 336
Ser Ser Asp Glu Asp Glu Tyr Glu Met Glu Ser Pro Asn Ile Thr Asp
70 75 80

ACC ATG AAG TTC TTT CTT TAT GTG CTT GAG TCT CTT CCA TCT CCC ACA 384
Thr Met Lys Phe Phe Leu Tyr Val Leu Glu Ser Leu Pro Ser Pro Thr
85 90 95 100

CTA ACT TGT GCA TTG ACT AAT GGA AGC ATT GAA GTC CAA TGC ATG ATA 432
Leu Thr Cys Ala Leu Thr Asn Gly Ser Ile Glu Val Gln Cys Met Ile
105 110 115

CCA GAG CAT TAC AAC AGC CAT CGA GGA CTT ATA ATG TAC TCA TGG GAT 480
Pro Glu His Tyr Asn Ser His Arg Gly Leu Ile Met Tyr Ser Trp Asp
120 125 130

TGT CCT ATG GAG CAA TGT AAA CGT AAC TCA ACC AGT ATA TAT TTT AAG 528
Cys Pro Met Glu Gln Cys Lys Arg Asn Ser Thr Ser Ile Tyr Phe Lys
135 140 145

ATG GAA AAT GAT CTT CCA CAA AAA ATA CAG TGT ACT CTT AGC AAT CCA 576
Met Glu Asn Asp Leu Pro Gln Lys Ile Gln Cys Thr Leu Ser Asn Pro
150 155 160

TTA TTT AAT ACA ACA TCA TCA ATC ATT TTG ACA ACC TGT ATC CCA AGC 624
Leu Phe Asn Thr Thr Ser Ser Ile Ile Leu Thr Thr Cys Ile Pro Ser
165 170 175 180

AGC GGT CAT TCA AGA CAC AGA TAT GCA CTT ATA CCC ATA CCA TTA GCA 672
Ser Gly His Ser Arg His Arg Tyr Ala Leu Ile Pro Ile Pro Leu Ala
185 190 195

GTA ATT ACA ACA TGT ATT GTG CTG TAT ATG AAT GGT ATT CTG AAA TGT 720
Val Ile Thr Thr Cys Ile Val Leu Tyr Met Asn Gly Ile Leu Lys Cys
200 205 210

GAC AGA AAA CCA GAC AGA ACC AAC TCC AAT TGA 753
Asp Arg Lys Pro Asp Arg Thr Asn Ser Asn
215 220

(2) INFORMATION FOR SEQ ID NO:2:

(i) SEQUENCE CHARACTERISTICS:
(A) LENGTH: 250 amino acids
(B) TYPE: amino acid
(D) TOPOLOGY: linear

(ii) MOLECULE TYPE: protein

(xi) SEQUENCE DESCRIPTION: SEQ ID NO:2:

Met Val Ala Gly Ser Asp Ala Gly Arg Ala Leu Gly Val Leu Ser Val
-28 -25 -20 -15

Val Cys Leu Leu His Cys Phe Gly Phe Ile Ser Cys Phe Ser Gln Gln
-10 -5 1

Ile Tyr Gly Val Val Tyr Gly Asn Val Thr Phe His Val Pro Ser Asn
5 10 15 20

Val Pro Leu Lys Glu Val Leu Trp Lys Lys Gln Lys Asp Lys Val Ala
                    25                  30                  35

Glu Leu Glu Asn Ser Glu Phe Arg Ala Phe Ser Ser Phe Lys Asn Arg
                40                  45                  50

Val Tyr Leu Asp Thr Val Ser Gly Ser Leu Thr Ile Tyr Asn Leu Thr
            55                  60                  65

Ser Ser Asp Glu Asp Glu Tyr Glu Met Glu Ser Pro Asn Ile Thr Asp
    70                  75                  80

Thr Met Lys Phe Phe Leu Tyr Val Leu Glu Ser Leu Pro Ser Pro Thr
85                  90                  95                  100

Leu Thr Cys Ala Leu Thr Asn Gly Ser Ile Glu Val Gln Cys Met Ile
                105                 110                 115

Pro Glu His Tyr Asn Ser His Arg Gly Leu Ile Met Tyr Ser Trp Asp
            120                 125                 130

Cys Pro Met Glu Gln Cys Lys Arg Asn Ser Thr Ser Ile Tyr Phe Lys
        135                 140                 145

Met Glu Asn Asp Leu Pro Gln Lys Ile Gln Cys Thr Leu Ser Asn Pro
    150                 155                 160

Leu Phe Asn Thr Thr Ser Ser Ile Ile Leu Thr Thr Cys Ile Pro Ser
165                 170                 175                 180

Ser Gly His Ser Arg His Arg Tyr Ala Leu Ile Pro Ile Pro Leu Ala
                185                 190                 195

Val Ile Thr Thr Cys Ile Val Leu Tyr Met Asn Gly Ile Leu Lys Cys
            200                 205                 210

Asp Arg Lys Pro Asp Arg Thr Asn Ser Asn
        215                 220

(2) INFORMATION FOR SEQ ID NO:3:

     (i) SEQUENCE CHARACTERISTICS:
          (A) LENGTH: 723 base pairs
          (B) TYPE: nucleic acid
          (C) STRANDEDNESS: single
          (D) TOPOLOGY: linear

    (ix) FEATURE:
          (A) NAME/KEY: CDS
          (B) LOCATION: 1..720

(ix) FEATURE:
(A) NAME/KEY: sig_peptide
(B) LOCATION: 1..84

(ix) FEATURE:
(A) NAME/KEY: mat_peptide
(B) LOCATION: 85..720

(ix) FEATURE:
(A) NAME/KEY: misc_feature
(B) LOCATION: 1..720
(D) OTHER INFORMATION: /note= "Human PI-linked LFA-3"

(ix) FEATURE:
(A) NAME/KEY: misc_feature
(B) LOCATION: 568..720
(D) OTHER INFORMATION: /note= "Signal sequence for
PI-linkage"

(xi) SEQUENCE DESCRIPTION: SEQ ID NO:3:

ATG GTT GCT GGG AGC GAC GCG GGG CGG GCC CTG GGG GTC CTC AGC GTG 48
Met Val Ala Gly Ser Asp Ala Gly Arg Ala Leu Gly Val Leu Ser Val
-28 -25 -20 -15

GTC TGC CTG CTG CAC TGC TTT GGT TTC ATC AGC TGT TTT TCC CAA CAA 96
Val Cys Leu Leu His Cys Phe Gly Phe Ile Ser Cys Phe Ser Gln Gln
-10 -5 1

ATA TAT GGT GTT GTG TAT GGG AAT GTA ACT TTC CAT GTA CCA AGC AAT 144
Ile Tyr Gly Val Val Tyr Gly Asn Val Thr Phe His Val Pro Ser Asn
5 10 15 20

GTG CCT TTA AAA GAG GTC CTA TGG AAA AAA CAA AAG GAT AAA GTT GCA 192
Val Pro Leu Lys Glu Val Leu Trp Lys Lys Gln Lys Asp Lys Val Ala
25 30 35

GAA CTG GAA AAT TCT GAA TTC AGA GCT TTC TCA TCT TTT AAA AAT AGG 240
Glu Leu Glu Asn Ser Glu Phe Arg Ala Phe Ser Ser Phe Lys Asn Arg
40 45 50

GTT TAT TTA GAC ACT GTG TCA GGT AGC CTC ACT ATC TAC AAC TTA ACA 288
Val Tyr Leu Asp Thr Val Ser Gly Ser Leu Thr Ile Tyr Asn Leu Thr
55 60 65

TCA TCA GAT GAA GAT GAG TAT GAA ATG GAA TCG CCA AAT ATT ACT GAT 336
Ser Ser Asp Glu Asp Glu Tyr Glu Met Glu Ser Pro Asn Ile Thr Asp
70 75 80

ACC ATG AAG TTC TTT CTT TAT GTG CTT GAG TCT CTT CCA TCT CCC ACA 384
Thr Met Lys Phe Phe Leu Tyr Val Leu Glu Ser Leu Pro Ser Pro Thr
85 90 95 100

CTA ACT TGT GCA TTG ACT AAT GGA AGC ATT GAA GTC CAA TGC ATG ATA 432
Leu Thr Cys Ala Leu Thr Asn Gly Ser Ile Glu Val Gln Cys Met Ile
105 110 115

CCA GAG CAT TAC AAC AGC CAT CGA GGA CTT ATA ATG TAC TCA TGG GAT 480
Pro Glu His Tyr Asn Ser His Arg Gly Leu Ile Met Tyr Ser Trp Asp
120 125 130

TGT CCT ATG GAG CAA TGT AAA CGT AAC TCA ACC AGT ATA TAT TTT AAG 528
Cys Pro Met Glu Gln Cys Lys Arg Asn Ser Thr Ser Ile Tyr Phe Lys
135 140 145

ATG GAA AAT GAT CTT CCA CAA AAA ATA CAG TGT ACT CTT AGC AAT CCA 576
Met Glu Asn Asp Leu Pro Gln Lys Ile Gln Cys Thr Leu Ser Asn Pro
150 155 160

TTA TTT AAT ACA ACA TCA TCA ATC ATT TTG ACA ACC TGT ATC CCA AGC 624
Leu Phe Asn Thr Thr Ser Ser Ile Ile Leu Thr Thr Cys Ile Pro Ser
165 170 175 180

AGC GGT CAT TCA AGA CAC AGA TAT GCA CTT ATA CCC ATA CCA TTA GCA 672
Ser Gly His Ser Arg His Arg Tyr Ala Leu Ile Pro Ile Pro Leu Ala
185 190 195

GTA ATT ACA ACA TGT ATT GTG CTG TAT ATG AAT GGT ATG TAT GCT TTT 720
Val Ile Thr Thr Cys Ile Val Leu Tyr Met Asn Gly Met Tyr Ala Phe
200 205 210

TAA 723

(2) INFORMATION FOR SEQ ID NO:4:

(i) SEQUENCE CHARACTERISTICS:
(A) LENGTH: 240 amino acids
(B) TYPE: amino acid
(D) TOPOLOGY: linear

(ii) MOLECULE TYPE: protein

(xi) SEQUENCE DESCRIPTION: SEQ ID NO:4:

Met Val Ala Gly Ser Asp Ala Gly Arg Ala Leu Gly Val Leu Ser Val
-28 -25 -20 -15

Val Cys Leu Leu His Cys Phe Gly Phe Ile Ser Cys Phe Ser Gln Gln
-10 -5 1

Ile Tyr Gly Val Val Tyr Gly Asn Val Thr Phe His Val Pro Ser Asn
  5                  10                  15                  20

Val Pro Leu Lys Glu Val Leu Trp Lys Lys Gln Lys Asp Lys Val Ala
                25                  30                  35

Glu Leu Glu Asn Ser Glu Phe Arg Ala Phe Ser Ser Phe Lys Asn Arg
            40                  45                  50

Val Tyr Leu Asp Thr Val Ser Gly Ser Leu Thr Ile Tyr Asn Leu Thr
        55                  60                  65

Ser Ser Asp Glu Asp Glu Tyr Glu Met Glu Ser Pro Asn Ile Thr Asp
    70                  75                  80

Thr Met Lys Phe Phe Leu Tyr Val Leu Glu Ser Leu Pro Ser Pro Thr
 85                  90                  95                 100

Leu Thr Cys Ala Leu Thr Asn Gly Ser Ile Glu Val Gln Cys Met Ile
                105                 110                 115

Pro Glu His Tyr Asn Ser His Arg Gly Leu Ile Met Tyr Ser Trp Asp
            120                 125                 130

Cys Pro Met Glu Gln Cys Lys Arg Asn Ser Thr Ser Ile Tyr Phe Lys
        135                 140                 145

Met Glu Asn Asp Leu Pro Gln Lys Ile Gln Cys Thr Leu Ser Asn Pro
    150                 155                 160

Leu Phe Asn Thr Thr Ser Ser Ile Ile Leu Thr Thr Cys Ile Pro Ser
165                 170                 175                 180

Ser Gly His Ser Arg His Arg Tyr Ala Leu Ile Pro Ile Pro Leu Ala
                185                 190                 195

Val Ile Thr Thr Cys Ile Val Leu Tyr Met Asn Gly Met Tyr Ala Phe
            200                 205                 210

(2) INFORMATION FOR SEQ ID NO:5:

     (i) SEQUENCE CHARACTERISTICS:
          (A) LENGTH: 1056 base pairs
          (B) TYPE: nucleic acid
          (C) STRANDEDNESS: single
          (D) TOPOLOGY: linear

    (ix) FEATURE:
          (A) NAME/KEY: CDS
          (B) LOCATION: 1..1053

(ix) FEATURE:
(A) NAME/KEY: sig_peptide
(B) LOCATION: 1..72

(ix) FEATURE:
(A) NAME/KEY: mat_peptide
(B) LOCATION: 73..1053

(ix) FEATURE:
(A) NAME/KEY: misc_feature
(B) LOCATION: 1..1053
(D) OTHER INFORMATION: /note= "Human CD2"

(ix) FEATURE:
(A) NAME/KEY: misc_feature
(B) LOCATION: 628..702
(D) OTHER INFORMATION: /note= "Transmembrane domain"

(xi) SEQUENCE DESCRIPTION: SEQ ID NO:5:

ATG AGC TTT CCA TGT AAA TTT GTA GCC AGC TTC CTT CTG ATT TTC AAT 48
Met Ser Phe Pro Cys Lys Phe Val Ala Ser Phe Leu Leu Ile Phe Asn
-24 -20 -15 -10

GTT TCT TCC AAA GGT GCA GTC TCC AAA GAG ATT ACG AAT GCC TTG GAA 96
Val Ser Ser Lys Gly Ala Val Ser Lys Glu Ile Thr Asn Ala Leu Glu
-5 1 5

ACC TGG GGT GCC TTG GGT CAG GAC ATC AAC TTG GAC ATT CCT AGT TTT 144
Thr Trp Gly Ala Leu Gly Gln Asp Ile Asn Leu Asp Ile Pro Ser Phe
10 15 20

CAA ATG AGT GAT GAT ATT GAC GAT ATA AAA TGG GAA AAA ACT TCA GAC 192
Gln Met Ser Asp Asp Ile Asp Asp Ile Lys Trp Glu Lys Thr Ser Asp
25 30 35 40

AAG AAA AAG ATT GCA CAA TTC AGA AAA GAG AAA GAG ACT TTC AAG GAA 240
Lys Lys Lys Ile Ala Gln Phe Arg Lys Glu Lys Glu Thr Phe Lys Glu
45 50 55

AAA GAT ACA TAT AAG CTA TTT AAA AAT GGA ACT CTG AAA ATT AAG CAT 288
Lys Asp Thr Tyr Lys Leu Phe Lys Asn Gly Thr Leu Lys Ile Lys His
60 65 70

CTG AAG ACC GAT GAT CAG GAT ATC TAC AAG GTA TCA ATA TAT GAT ACA 336
Leu Lys Thr Asp Asp Gln Asp Ile Tyr Lys Val Ser Ile Tyr Asp Thr
75 80 85

AAA GGA AAA AAT GTG TTG GAA AAA ATA TTT GAT TTG AAG ATT CAA GAG 384
Lys Gly Lys Asn Val Leu Glu Lys Ile Phe Asp Leu Lys Ile Gln Glu
90 95 100

AGG GTC TCA AAA CCA AAG ATC TCC TGG ACT TGT ATC AAC ACA ACC CTG 432
Arg Val Ser Lys Pro Lys Ile Ser Trp Thr Cys Ile Asn Thr Thr Leu
105 110 115 120

ACC TGT GAG GTA ATG AAT GGA ACT GAC CCC GAA TTA AAC CTG TAT CAA 480
Thr Cys Glu Val Met Asn Gly Thr Asp Pro Glu Leu Asn Leu Tyr Gln
125 130 135

GAT GGG AAA CAT CTA AAA CTT TCT CAG AGG GTC ATC ACA CAC AAG TGG 528
Asp Gly Lys His Leu Lys Leu Ser Gln Arg Val Ile Thr His Lys Trp
140 145 150

ACC ACC AGC CTG AGT GCA AAA TTC AAG TGC ACA GCA GGG AAC AAA GTC 576
Thr Thr Ser Leu Ser Ala Lys Phe Lys Cys Thr Ala Gly Asn Lys Val
155 160 165

AGC AAG GAA TCC AGT GTC GAG CCT GTC AGC TGT CCA GAG AAA GGT CTG 624
Ser Lys Glu Ser Ser Val Glu Pro Val Ser Cys Pro Glu Lys Gly Leu
170 175 180

GAC ATC TAT CTC ATC ATT GGC ATA TGT GGA GGA GGC AGC CTC TTG ATG 672
Asp Ile Tyr Leu Ile Ile Gly Ile Cys Gly Gly Gly Ser Leu Leu Met
185 190 195 200

GTC TTT GTG GCA CTG CTC GTT TTC TAT ATC ACC AAA AGG AAA AAA CAG 720
Val Phe Val Ala Leu Leu Val Phe Tyr Ile Thr Lys Arg Lys Lys Gln
205 210 215

AGG AGT CGG AGA AAT GAT GAG GAG CTG GAG ACA AGA GCC CAC AGA GTA 768
Arg Ser Arg Arg Asn Asp Glu Glu Leu Glu Thr Arg Ala His Arg Val
220 225 230

GCT ACT GAA GAA AGG GGC CGG AAG CCC CAC CAA ATT CCA GCT TCA ACC 816
Ala Thr Glu Glu Arg Gly Arg Lys Pro His Gln Ile Pro Ala Ser Thr
235 240 245

CCT CAG AAT CCA GCA ACT TCC CAA CAT CCT CCT CCA CCA CCT GGT CAT 864
Pro Gln Asn Pro Ala Thr Ser Gln His Pro Pro Pro Pro Pro Gly His
250 255 260

CGT TCC CAG GCA CCT AGT CAT CGT CCC CCG CCT CCT GGA CAC CGT GTT 912
Arg Ser Gln Ala Pro Ser His Arg Pro Pro Pro Pro Gly His Arg Val
265 270 275 280

CAG CAC CAG CCT CAG AAG AGG CCT CCT GCT CCG TCG GGC ACA CAA GTT 960
Gln His Gln Pro Gln Lys Arg Pro Pro Ala Pro Ser Gly Thr Gln Val
285 290 295

CAC CAG CAG AAA GGC CCG CCC CTC CCC AGA CCT CGA GTT CAG CCA AAA 1008
His Gln Gln Lys Gly Pro Pro Leu Pro Arg Pro Arg Val Gln Pro Lys
300 305 310

CCT CCC CAT GGG GCA GCA GAA AAC TCA TTG TCC CCT TCC TCT AAT 1053
Pro Pro His Gly Ala Ala Glu Asn Ser Leu Ser Pro Ser Ser Asn
315 320 325

TAA 1056

(2) INFORMATION FOR SEQ ID NO:6:

(i) SEQUENCE CHARACTERISTICS:
(A) LENGTH: 351 amino acids
(B) TYPE: amino acid
(D) TOPOLOGY: linear

(ii) MOLECULE TYPE: protein

(xi) SEQUENCE DESCRIPTION: SEQ ID NO:6:

Met Ser Phe Pro Cys Lys Phe Val Ala Ser Phe Leu Leu Ile Phe Asn
-24 -20 -15 -10

Val Ser Ser Lys Gly Ala Val Ser Lys Glu Ile Thr Asn Ala Leu Glu
-5 1 5

Thr Trp Gly Ala Leu Gly Gln Asp Ile Asn Leu Asp Ile Pro Ser Phe
10 15 20

Gln Met Ser Asp Asp Ile Asp Asp Ile Lys Trp Glu Lys Thr Ser Asp
25 30 35 40

Lys Lys Lys Ile Ala Gln Phe Arg Lys Glu Lys Glu Thr Phe Lys Glu
45 50 55

Lys Asp Thr Tyr Lys Leu Phe Lys Asn Gly Thr Leu Lys Ile Lys His
60 65 70

Leu Lys Thr Asp Asp Gln Asp Ile Tyr Lys Val Ser Ile Tyr Asp Thr
75 80 85

Lys Gly Lys Asn Val Leu Glu Lys Ile Phe Asp Leu Lys Ile Gln Glu
90 95 100

Arg Val Ser Lys Pro Lys Ile Ser Trp Thr Cys Ile Asn Thr Thr Leu
105 110 115 120

Thr Cys Glu Val Met Asn Gly Thr Asp Pro Glu Leu Asn Leu Tyr Gln
125 130 135

Asp Gly Lys His Leu Lys Leu Ser Gln Arg Val Ile Thr His Lys Trp
140 145 150

Thr Thr Ser Leu Ser Ala Lys Phe Lys Cys Thr Ala Gly Asn Lys Val
        155                 160                 165
Ser Lys Glu Ser Ser Val Glu Pro Val Ser Cys Pro Glu Lys Gly Leu
    170                 175                 180
Asp Ile Tyr Leu Ile Ile Gly Ile Cys Gly Gly Gly Ser Leu Leu Met
185                 190                 195                 200
Val Phe Val Ala Leu Leu Val Phe Tyr Ile Thr Lys Arg Lys Lys Gln
                205                 210                 215
Arg Ser Arg Arg Asn Asp Glu Glu Leu Glu Thr Arg Ala His Arg Val
            220                 225                 230
Ala Thr Glu Glu Arg Gly Arg Lys Pro His Gln Ile Pro Ala Ser Thr
        235                 240                 245
Pro Gln Asn Pro Ala Thr Ser Gln His Pro Pro Pro Pro Pro Gly His
    250                 255                 260
Arg Ser Gln Ala Pro Ser His Arg Pro Pro Pro Pro Gly His Arg Val
265                 270                 275                 280
Gln His Gln Pro Gln Lys Arg Pro Pro Ala Pro Ser Gly Thr Gln Val
                285                 290                 295
His Gln Gln Lys Gly Pro Pro Leu Pro Arg Pro Arg Val Gln Pro Lys
            300                 305                 310
Pro Pro His Gly Ala Ala Glu Asn Ser Leu Ser Pro Ser Ser Asn
        315                 320                 325